# EUROPÄISCHE PATENTSCHRIFT

(11) **EP 1 341 897 B1**
(45) Veröffentlichungstag und Bekanntmachung des Hinweises auf die Patenterteilung: **29.06.2005**
(21) Anmeldenummer: 01991823.4
(22) Anmeldetag: 11.12.2001
(51) Int. Cl.: C12M 1/24, B01L 3/00, C12Q 1/68

(54) **IN SITU-HYBRIDISIERUNGS-ANORDNUNG ZUM SPEZIFISCHEN NACHWEIS VON MIKROORGANISMEN**
IN SITU HYBRIDIZATION SYSTEM FOR SPECIFICALLY DETECTING MICROORGANISMS
SYSTEME D'HYBRIDATION IN SITU DE MISE EN EVIDENCE SPECIFIQUE DE MICRO-ORGANISMES

(30) Priorität: 11.12.2000 DE 10061655
(43) Veröffentlichungstag der Anmeldung: 10.09.2003
(73) Patentinhaber: Vermicon AG, 80992 München (DE)
(72) Erfinder: MÜHLHAHN, Peter, 80992 München (DE); SNAIDR, Jiri, 80992 München (DE)
(74) Vertreter: Bohmann, Armin K., Dr.
(86) Internationale Anmeldenummer: PCT/EP2001/014543
(87) Internationale Veröffentlichungsnummer: WO 2002/048398

(56) Entgegenhaltungen:
- EP-A- 0 298 045
- EP-A- 0 497 464
- EP-A- 0 673 679
- WO-A-00/65093
- WO-A-96/31626
- WO-A-98/15656
- FR-A- 2 525 233
- US-A- 3 616 265
- US-A- 5 192 503
- US-A- 5 380 492
- SNAIDR J ET AL: "APPLICATION OF THE 16S RRNA APPROACH TO INDUSTRIAL SEWAGE TREATMENT PLANTS: PROGRESS TOWARDS A COMPLETE COMMUNITY ANALYSIS" MEDEDELINGEN VAN DE FACULTEIT LANDBOUWWETENSCHAPPEN UNIVERSITEIT GENT, GENT, BE, Bd. 63, Nr. 4B, 1998, Seiten 1741-1746, XP000982316 ISSN: 0368-9697

## Beschreibung

Die Erfindung betrifft eine in situ-Hybridisierungs-Anordnung zum spezifischen Nachweis von Mikroorganismen, ein Verfahren zum spezifischen Nachweis von Mikroorganismen durch in situ-Hybridisierung sowie einen Kit, mit dem die Identifizierung und Visualisierung von Mikroorganismen in einer Probe möglich ist.

Moderne Methoden der Bakterienbestimmung haben alle ein gemeinsames Ziel: Sie versuchen die Nachteile der Kultivierung zu umgehen, indem sie keine Kultivierung der Bakterien mehr benötigen.

Bei der PCR, der Polymerase-Kettenreaktion wird mit bakterienspezifischen Primern ein bestimmtes charakteristisches Stück des Bakteriengenoms amplifiziert. Findet der Primer seine Zielstelle, so kommt es zu einer millionenfachen Vermehrung eines Stücks der Erbsubstanz. Bei der anschließenden Analyse mittels eines DNA-Fragmente auftrennenden Agarose-Gels kann eine qualitative Bewertung stattfinden. Im einfachsten Fall führt dies zu der Aussage, dass die Zielstellen in der untersuchten Probe vorhanden sind. Keine weiteren Aussagen sind zulässig, da die Zielstellen von einem lebenden Bakterium, von einem toten Bakterium oder von nackter DNA stammen können. Eine Differenzierung ist hier nicht möglich. Eine Weiterführung dieser Technik stellt die quantitative PCR dar, bei der versucht wird, eine Korrelation zwischen Menge an vorhandenen Bakterien und Menge an erhaltener und amplifizierter DNA herzustellen.

Doch auch biochemische Parameter werden zur Bakterienidentifizierung verwendet: So dient die Erstellung von Bakterienprofilen auf der Basis von Quinonbestimmungen dazu, ein möglichst verzerrungsfreies Bild der Bakterienpopulation wiederzugeben (Hiraishi, A. 1988. Respiratory quinone profiles as tools for identifying differnet bacterial populations in activated sludge. J. Gen. Appl. Microbiol. 34:39-56). Doch auch diese Methode ist von der Kultivierung einzelner Bakterien abhängig, da zur Erstellung der Referenzdatenbank die Quinonprofile der Bakterien in Reinkultur benötigt werden. Überdies vermag die Bestimmung der Bakterienquinonprofile auch keinen wirklichen Eindruck davon zu vermitteln, welche tatsächlichen Populationsverhältnisse in der Probe vorliegen.

Die Detektion der Bakterien mit Antikörpern ist im Unterschied hierzu eine direktere Methode (Brigmon, R. L., G. Bitton, S. G. Zam, and B. O'Brien. 1995. Development and application of a monoclonal antibody against Thiothrix spp. Appl. Environ. Microbiol. 61:13-20). Fluoreszenzmarkierte Antikörper werden mit der Probe vermengt und erlauben ein hochspezifisches Anheften an die bakteriellen Antigene. Im Epifluoreszenz-Mikroskop werden die Bakterien anhand ihrer emittierten Fluoreszenz anschließend detektiert. Auf diese Weise können Bakterien bis auf Stammebene identifiziert werden. Drei entscheidende Nachteile schränken aber die Anwendbarkeit dieser Methode empfindlich ein: Erstens werden für die Herstellung der Antikörper wiederum Reinkulturen benötigt. Zweitens bereitet der oftmals großvolumige und sperrige Antikörper-Fluoreszenzmolekülkomplex Probleme beim Eindringen in die Zielzellen. Drittens ist die Detektion oftmals zu spezifisch. Die in der Produktion teuren Antikörper detektieren spezifisch oft nur einen bestimmten Bakterienstamm, lassen aber andere Stämme der gleichen Bakterienart unentdeckt. Oft ist aber er keine stammspezifische Detektion der Bakterien notwendig, sondern vielmehr die Detektion einer Bakterienart oder einer ganzen Bakteriengruppe. Viertens ist die Produktion der Antikörper ein relativ langwieriger und teurer Vorgang.

Einen einzigartigen Ansatz die Spezifität der molekularbiologischen Methoden wie der PCR mit der Möglichkeit der Bakterienvisualisierung, wie sie die Antikörper-Methode ermöglicht, zu verbinden, bietet die Methode der Fluoreszenz-In-Situ-Hybridisierung (FISH; Amann, R. I., W. Ludwig, and K.-H. Schleifer. 1995. Phylogenetic identification and in situ detection of individual microbial cells without cultivation. Microbial. Rev. 59:143-169). Hierbei können Bakterienarten, -gattungen oder -gruppen hochspezifisch direkt in der Probe visualisiert und identifiziert werden. Diese Methode ist der einzige Ansatz, der eine verzerrungsfreie Darstellung der tatsächlichen in situ-Verhältnisse der Biozönose darstellt. Sogar bisher nichtkultivierte und demnach nicht beschriebene Bakterien können identifiziert und zusätzlich auch direkt in der Probe visualisiert werden.

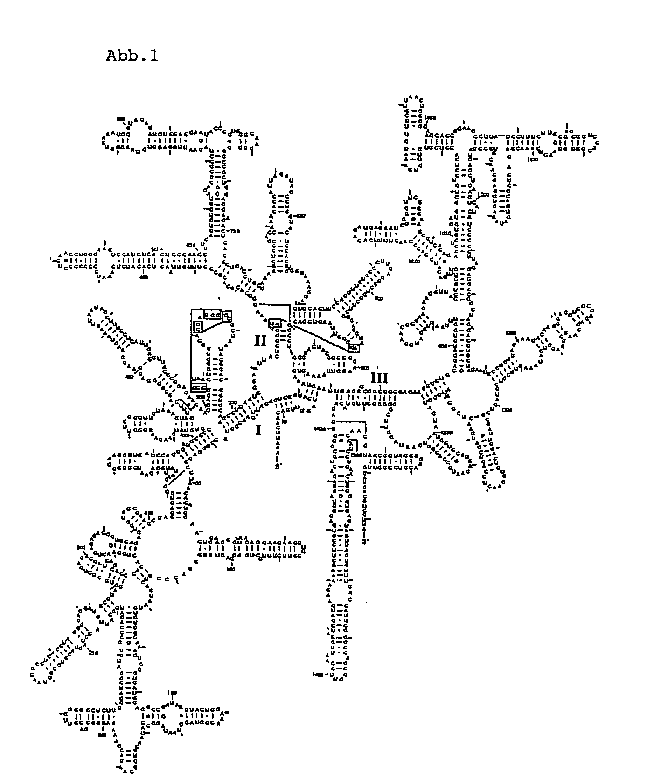

Die FISH-Technik basiert auf der Tatsache, dass es in Bakterienzellen bestimmte Moleküle gibt, die aufgrund ihrer lebenswichtigen Funktion im Laufe der Evolution nur wenig mutiert wurden: Die 16S und die 23S ribosomale Ribonukleinsäure (rRNS). Beide sind Bestandteile der Ribosomen, den Orten der Proteinbiosynthese, und können aufgrund ihrer ubiquitären Verbreitung, ihrer Größe, und ihrer strukturellen und funktionellen Konstanz als phylogenetische Marker dienen (Woese, C. R. 1987. Bacterial evolution. Microbiol. Rev. 51:221-271). Ausgehend von einer vergleichenden Sequenzanalyse können phylogenetische Beziehungen allein aufgrund dieser Daten aufgestellt werden. Dazu müssen diese Sequenzdaten in ein Alignment gebracht werden. Im Alignment, welches sich auf Kenntnisse über die Sekundärstruktur und Tertiärstruktur dieser Makromoleküle stützt, werden die homologen Positionen der ribosomalen Nukleinsäuren in Einklang miteinander gebracht. Abbildung 1 zeigt das Sekundärstrukturmodell einer 16S rRNA.

Ausgehend von diesen Daten können phylogenetische Berechnungen durchgeführt werden. Der Einsatz modernster Computertechnologie macht es möglich, auch großangelegte Berechnungen schnell und effektiv auszuführen, sowie große Datenbanken, welche die Alignment-Sequenzen der 16S-rRNA und 23S-rRNA beinhalten, anzulegen. Durch den schnellen Zugriff auf dieses Datenmaterial können neu erhaltene Sequenzen in kurzer Zeit phylogenetisch analysiert werden. Diese rRNA Datenbanken können dazu verwendet werden, spezifische Gensonden zu konstruieren. Hierbei werden alle-verfügbaren rRNA Sequenzen miteinander verglichen und für bestimmte Sequenzstellen Sonden entworfen, die spezifisch eine Bakterienart, -gattung oder -gruppe erfassen.

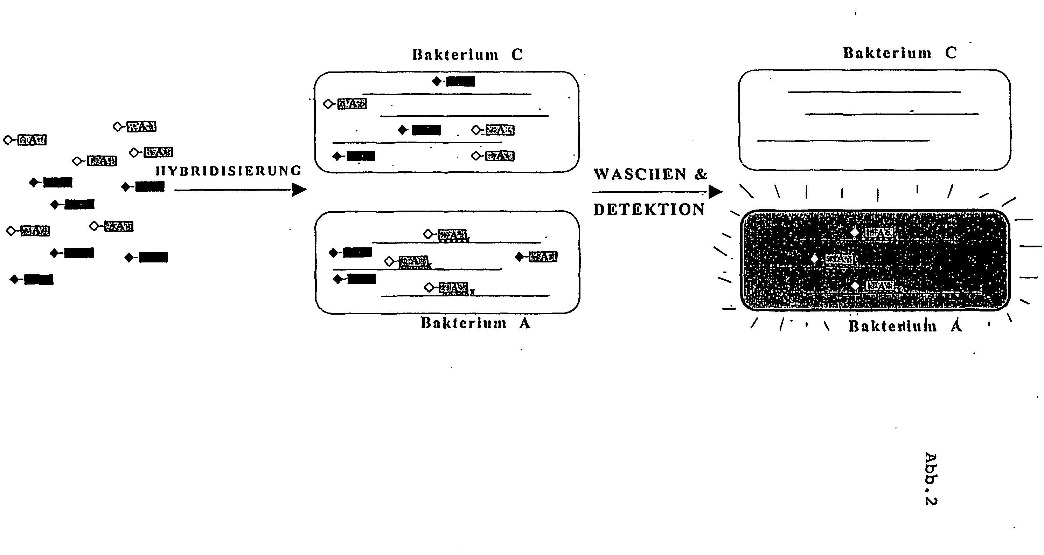

Bei der FISH (Fluoreszenz-In-Situ-Hybridisierung)-Technik werden diese Gensonden, die zu einer bestimmten Region auf der ribosomalen Zielsequenz komplementär sind, in die Zelle geschleust. Die Gensonden sind i.d.R. kleine, 16-20 Basen lange, einzelsträngige Desoxyribonukleinsäurestücke und richten sich gegen eine Zielregion, welche typisch für eine Bakterienart oder eine Bakteriengruppe ist. Findet die fluoreszenzmarkierte Gensonde in einer Bakterienzelle ihre Zielsequenz, so bindet sie daran und die Zellen können aufgrund ihrer Fluoreszenz im Fluoreszenzmikroskop detektiert werden. Abbildung 2 verdeutlicht den Ablauf einer in situ-Hybridisierung.

Kultivierungsabhängige Verfahren liefern nur einen sehr verfälschten Einblick in die Zusammensetzung und Dynamik der mikrobiellen Biozönose. Durch die FISH Technik konnte gezeigt werden, daß es z.B. bei der Erfassung der Belebtschlammflora durch Kultivierung zu einem Kultivierungsshift kommt (Wagner, M., R. Amann, H. Lemmer, and K.H. Schleifer. 1993. Probing activated sludge with oligonucleotides specific for proteobacteria: inadequacy of culturedependent methods for describing microbial community structure. Appl. Environ. Microbiol. 59:1520-1525).

Durch diese mediumabhängige Verzerrung der realen Verhältnisse innerhalb der Bakterienpopulation werden Bakterien, die im Belebtschlamm eine untergeordnete Rolle spielen, aber den verwendeten Kultivierungsbedingungen gut angepaßt sind, in ihrer Bedeutung dramatisch überschätzt. So konnte gezeigt werden, dass aufgrund eines solchen Kultivierungsartefaktes die Bakteriengattung *Acinetobacter* bezüglich ihrer Rolle als biologischer Phosphatentferner in der Abwasserreinigung völlig falsch eingeschätzt wurde. Als Folge solcher Fehlbewertungen entstehen kostenintensive, fehlerbehaftete bzw. unpräzise Modellierungen von Anlagen. Die Effizienz und Reproduzierbarkeit solcher Simulationsberechnungen ist gering.

Die Vorteile der FISH-Technik gegenüber der Bakterienidentifizierung mittels Kultivierung sind vielfältig. Erstens können mit Gensonden sehr viel mehr Zellen detektiert werden. Während durch die Kultivierung maximal nur 15% der Bakterienpopulation einer Probe sichtbar gemacht werden können, erlaubt die FISH Technik in vielen Proben eine Detektion bis zu 100% der Bakteriengesamtpopulation. Zweitens kann der aktive Anteil einer Population durch das Verhältnis der gegen alle Bakterien gerichteten Sonde und einer unspezifischen Zellfärbung bestimmt werden. Drittens werden die Bakterien direkt am Ort ihres Wirkens (in situ) sichtbar gemacht. Mögliche Wechselwirkungen zwischen verschiedenen Bakterienpopulationen können so erkannt und analysiert werden. Viertens ist die Detektion von Bakterien mittels der FISH-Technik sehr viel schneller als mittels Kultivierung. Benötigt die Identifizierung von Bakterien mittels Kultivierung oft mehrere Tage, so vergehen von der Probennahme bis zur Bakterienidentifizierung sogar auf Artebene mittels der FISH-Technik nur wenige Stunden. Fünftens können Gensonden in ihrer Spezifität fast beliebig frei gewählt werden. Einzelne Arten können genauso gut mit einer Sonde erfasst werden, wie ganze Gattungen oder Bakteriengruppen. Sechstens können Bakterienarten oder ganze Bakterienpopulationen direkt in der Probe exakt quantifiziert werden. Der Umweg über eine Kultivierung und eine damit verbundene nur unzureichende Quantifizierung muss nicht gegangen werden.

Wird ein in einer Probe vorkommendes Bakterium auf seine Taxonomie hin untersucht, so wird der top-to-bottom Approach angewandt Hierbei wird die Bakterienprobe zu Beginn der Untersuchung mit möglichst breiten, d.h. wenig spezifischen und nur ganze Bakteriengruppen erfassenden Gensonden analysiert. Eine sukzessive Erhöhung der Spezifität der eingesetzten Sonden erlaubt schließlich die Identifizierung des unbekannten Bakteriums.

Die FISH-Technik ist demnach ein überragendes Werkzeug, um Bakterien schnell und äußerst spezifisch direkt in einer Probe nachzuweisen. Sie ist im Gegensatz zu Kultivierungsverfahren eine direkte Methode und erlaubt gegenüber modernen Verfahren nicht nur die Visualisierung der Bakterien, sondern darüber hinaus auch deren exakte Quantifizierung.

Die FISH-Analyse wird grundsätzlich auf einem Objektträger durchgeführt, da bei der Auswertung die Bakterien durch Bestrahlung mit einem hochenergetischen Licht visualisiert, also sichtbar gemacht werden. Die Zusammensetzung der einzelnen Lösungen wie Hybridisierungspuffer bzw. Hybridisierungslösung und Waschpuffer bzw. Waschlösung ist dem Fachmann wohlbekannt und wird z.B. in Snaidr et. al, 1997 (J. Snaidr, R. Amann, I. Huber, W. Ludwig, and K.-H. Schleifer. 1997. Phylogenetic analysis and in situ identification of bacteria in activated sludge. Appl. Env. Microb. 63:7, 2884-2896) ausführlich beschrieben. Die Durchführung der FISH für die Analyse von Mikroorganismen auf einem Objektträger umfasst üblicherweise die im folgenden dargestellten Schritte.
1. Einbringen eines Aliquots der mikrobiellen Probe in ein Reaktionsgefäß und Vermischen mit einer geeigneten Fixierungslösung.
2. Mehrere Zentrifugations- und Waschschritte bis die Probe fixiert und damit für Gensonden zugänglich ist.
3. Aufbringen eines Aliquots der fixierten mikrobiellen Probe in eine Aussparung auf dem Objekträger.
4. Eintrocknen der Mikroorganismen auf dem Objektträger durch Inkubation in einem Ofen bei 40 - 90°C und 10 - 30 min.
5. Dehydratisierung der mikrobiellen Zellen in einer aufsteigenden Ethanolreihe: Hierbei wird der Objektträger nacheinander in eine 50%ige, 70%ige und 100%ige Ethanollösung eingetaucht.
6. Aufbringen einer Hybridisierungslösung auf die Aussparung, welche die Mikroorganismen beinhaltet.
7. Aufbringen einer Sondenlösung auf dieselbe Aussparung.
8. Vorbereitung einer feuchten Kammer: Hierbei wird ein Stück Zellstoff gefaltet in ein Plastikröhrchen eingeführt. Der Zellstoff wird dann in der Kammer liegend mit mehreren ml der Hybridisierungslösung befeuchtet
9. Der Objektträger wird waagrecht auf den Zellstoff in die feuchte Kammer gelegt.
10. Die feuchte Kammer wird in einen Inkubationsofen eingebracht und für 1-2 Stunden inkubiert.
11. Die feuchte Kammer wird geöffnet, der Objektträger wird herausgenommen und kurz mit destillierten Wasser gespült.
12. Die feuchte Kammer wird verworfen.
13. Eine Waschpufferlösung wird in ein neues Plastikröhrchen eingeführt.
14. Der gespülte Objektträger wird in das mit der Waschlösung gefüllte Plastikröhrchen eingeführt.
15. Der Objektträger wird in dem Plastikröhrchen in einem Inkubationsofen für 10-30 min inkubiert.
16. Der Objektträger wird aus dem Plastikröhrchen herausgenommen und mit destillierten Wasser gewaschen.
17. Der Objektträger wird in schräger Stellung luftgetrocknet.
18. Nach Auftragen von einem Antifadingreagenz auf den Objektträger, kann dieser unter einem Epifluroeszenz-Mikroskop betrachtet werden.

Das vorstehend beschriebene herkömmliche FISH-Verfahren zum Nachweis von Mikroorganismen weist jedoch wesentliche Nachteile auf. So ist es aufwendig, langwierig und aufgrund verschiedener Fehlerquellen nicht mit gleichbleibender Qualität reproduzierbar. Dies wird im folgenden ausführlich dargestellt.

So kann die Fixierung der Probe aufgrund der zahlreichen Wasch- und Zentrifugationsschritte unterschiedlich effizient verlaufen. Dies führt im nachfolgenden Hybridisierungsvorgang zu einem unterschiedlich effizienten Eindringen der Gensonden in die Zellen und deshalb zu unterschiedlich Helligkeiten bei der Detektion der Zellen unter dem Epifluoreszenz-Mikroskop. Ursächlich korreliert die Helligkeit aber mit dem Ribosomengehalt der Zellen. Daher ist z.B. bei der FISH-Analyse die Intensität der Fluoreszenz ein Maßstab dafür, ob die Zellen sich zum Zeitpunkt der Probennahme in einem guten oder in einem schlechten Wachstumszustand befanden. Diese Aussage ist für eine Gesamtbeurteilung des mikrobiellen Zustandes einer Probe gerade in der medizinischen Mikrobiologie, aber auch in der Lebensmittel- oder Umweltmikrobiologie von Bedeutung. Folglich führt eine unterschiedlich effiziente Fixierung der zu untersuchenden Probe zu verfälschten Aussagen über den Wachstumszustand und damit über den mikrobiellen Gesamtzustand einer Probe.

Ferner mindert eine schlechte Signalintensität aufgrund einer ineffizienten Fixierung die Fähigkeit des Betrachters, auch kleine Zellen oder Zellen mit einem niedrigen Ribosomengehalt bei der Betrachtung im Epifluoreszenz-Mikroskop zu detektieren. Beim Fixierungsvorgang in Reaktionsgefäßen können weiterhin durch verschiedene Wasch- und Zentrifugationsschritte Zellen verloren gehen.

Ein weiteres Problem des herkömmlichen FISH-Verfahrens ist, daß sich bei der Dehydratisierung der Zellen durch mehrere Inkubationen Zellen vom Objektträger lösen bzw. auf andere Aussparungen übertragen werden können.

Ferner führt die Trennung von Sondenlösung und Hybridisierungslösung zu einem höheren Arbeitsaufwand, da zwei verschiedene Lösungen auf die Objektträgeraussparung pipettiert werden müssen.

Des weiteren ist die Vorbereitung der feuchten Kammer umständlich und garantiert keine horizontale Lage des Objektträgers. Dies kann unter Umständen zu einem Vermischen der sich auf den verschiedenen Aussparungen befindlichen Lösungen führen.

Ein weiteres Problem stellt bei Verwendung eines in der Literatur zitierten runden Plastikröhrchens als Hybridisierungskammer die in der Regel geringe Standfestigkeit der feuchten Kammer im Inkubationsofen dar. Diese geringe Standfestigkeit kann zu einer Destabilisierung des Objektträgers und zu einem Vermischen der Lösungen der verschiedenen Objektträgeraussparungen führen.

Ferner muss der Objektträger beim Waschvorgang zuerst gespült und dann in einen anderen Behälter überführt werden. Durch diesen relativ langwierigen Vorgang können durch die erniedrigten Hybridisierungstemperaturen unspezifische Bindungen von Nukleinsäuresondenmolekülen an die Zellen stattfinden.

Weitere Probleme der herkömmlichen FISH-Verfahren sind, dass beim Waschvorgang mit destilliertem Wasser Zellen heruntergespült oder in eine andere Obj ektträgeraussparung gespült werden können, und dass bei der Lufttrocknung in senkrechter Stellung durch herablaufende Tropfen Zellen von einer Objektträgeraussparung in die nächste übertragen werden können. Schließlich kann das unsichere Auflegen des Objektträgers zum Umstürzen und damit zu einem Ablösen der Zellen führen.

Die schlechte Reproduzierbarkeit und die aufwendige, langwierige und umständliche Handhabung haben dazu geführt, dass die in situ-Hybridisierung im allgemeinen und insbesondere die FISH-Analyse bislang in der Industrie kaum verwendet werden. Da die Analyse von Bakterien mittels dieser Verfahren jedoch bedeutende Vorteile gegenüber allen anderen heutzutage industriell verwendeten mikrobiologischen Analyseverfahren besitzt, besteht ein Bedarf an einer Vorrichtung bzw. einem Verfahren, mit dem eine einfache und reproduzierbare Identifizierung von Mikroorganismen durch in situ-Hybridsierung und insbesondere durch das FISH-Verfahren möglich ist.

Aufgabe der vorliegenden Erfindung ist es somit, die vorstehend beschriebenen Nachteile des Stands der Technik zu überwinden und eine Vorrichtung bzw. eine Anordnung sowie ein Verfahren bereitzustellen, mit dem eine schnelle Identifizierung von Mikroorganismen in einer Probe einfach und reproduzierbar durchgeführt werden kann.

US-A-5 192 503 offenbart eine Hybridisierungsanordnung umfassend einen mit mindestens einer Öffnung versehener Behälter, einen Träger für eine Reaktionslösung, einen Objektträger, Befestigungsmittel für den Objektträger und einen Deckel.

Weitere Aufgaben ergeben sich aus der nachfolgenden Beschreibung der Erfindung.

Oben genannte Aufgaben werden erfindungsgemäß durch die Merkmale der unabhängigen Ansprüche gelöst. Weitere Ausführungsformen ergeben sich aus den Merkmalen der Unteransprüche.

Erfindungsgemäß wird eine in situ-Hybridisierungs-Anordnung zum spezifischen Nachweis von Mikroorganismen bereitgestellt, die einen mit mindestens einer Öffnung (2) versehenen Behälter (1); einen Träger für die Hybridisierungslösung (3); einen Objektträger (4); und Befestigungsmittel (5) für den Objektträger umfasst. Weiterhin umfasst die Anordnung einen Deckel (6), der zum dichten Abschließen, insbesondere zum wasser und/oder luftdichten Abschließen der Öffnung des Behälters geeignet ist. Der Begriff "dicht" ist in diesem Zusammenhang so zu verstehen, dass in dem Behälter vorhandene Feuchtigkeit in geschlossenem Zustand im wesentlichen nicht aus dem Behälter entweicht.

Der Objektträger ist vorzugsweise mit Aussparungen (9) versehen, in die die zu untersuchende Probe und gegebenenfalls Negativ- bzw. Positivproben getrennt voneinander aufgetragen werden können. Besonders bevorzugt sind die Aussparungen auf dem Objektträger nur in einer Dimension von weiteren Aussparungen benachbart, also z.B. in einer Reihe angeordnet, wobei die Aussparungen innerhalb der Reihe auch versetzt sein können.

Bei der erfindungsgemäßen in situ-Hybridisierungs-Anordnung umfasst der Deckel die Befestigungsmittel (5) für den Objektträger. Besonders bevorzugt umfasst der Deckel als Befestigungsmittel für den Objektträger einen Schlitz (5), in den ein Ende (7) des Objektträgers gesteckt werden kann. Alternativ kann der Objektträger natürlich auch in Befestigungsmittel (5) eingreifen, die Bestandteil des Behälters sind und z.B. in Form eines Schlitzes im Boden des Behälters vorliegen, wobei der Boden in diesem Fall der Öffnung des Behälters gegenüber liegt.

Bei einer bevorzugten Ausführungsform der erfindungsgemäßen Anordnung ist der Deckel mit einem Bauteil (8) versehen, welches eine stabile Standlage des Deckels mit befestigtem Objektträger getrennt von der Anordnung ohne Deckel bei horizontaler Stellung des Objektträgers ermöglicht.

Besonders bevorzugt ist der Deckel so konstruiert, dass das Stehen des Deckels mit befestigtem Objektträger getrennt von der Anordnung ohne Deckel bei horizontaler bzw. vertikaler bzw. seitlicher Stellung des Objektträgers möglich ist.

Unter horizontaler Stellung des Objektträgers wird im Rahmen der vorliegenden Erfindung verstanden, dass die Stellung des Objektträgers derart ist, dass das Auftragen von Proben bzw. Sonden auf den Objektträger ohne Verfließen möglich ist.

Unter seitlicher Stellung des Objektträgers wird im Rahmen der vorliegenden. Erfindung eine gegenüber der horizontalen Stellung um 90° gedrehte Stellung verstanden, wobei der Objektträger derart um 90° gedreht ist, dass Tropfen vom Objektträger ablaufen können, ohne gegebenenfalls in eine andere Aussparung auf dem Objektträger hineinzulaufen, unter der Maßgabe, dass die Aussparungen nur in einer Dimension von weiteren Aussparungen benachbart sind.

Unter vertikaler Stellung des Objektträgers wird im Rahmen der vorliegenden Erfindung eine sowohl gegenüber der horizontalen als auch gegenüber der seitlichen Stellung um 90° gedrehte Stellung verstanden.

Bei einer besonders bevorzugten Ausführung der erfindungsgemäßen Anordnung sind der Behälter und/oder der Deckel so konstruiert, dass sowohl bei nicht geschlossenem Deckel als auch bei geschlossenem Deckel eine stabile Standlage der Anordnung bei horizontaler bzw. vertikaler bzw. seitlicher Stellung des Objektträgers ermöglicht ist.

Erfindungsgemäß bevorzugt ist ferner eine Anordnung, bei der seitliche Führungsschienen (10) für den Objektträger an dem Behälter zur Stabilisierung des Objektträgers in dem Behälter angebracht sind bzw. solche Führungsschienen (10) Bestandteil des Behälters sind.

Des weiteren ist der Hybridisierungslösungs-Träger (3) vorzugsweise herausnehmbar bzw. einsetzbar. Erfindungsgemäß bevorzugt ist ferner, dass der Träger für die Hybridisierungslösung (3) vollständig in den Behälter eingebracht werden kann. Vorzugsweise kann der Träger für die Hybridisierungslösung (3) aber auch teilweise, insbesondere stufenlos, in den Behälter (1) eingebracht werden.

Bei einer weiteren bevorzugten Ausführungsform sind seitliche Führungsschienen (11) für den Hybridisierungslösungs-Träger (3) an dem Behälter (1) zur Stabilisierung des Hybridisierungslösungs-Trägers (3) in dem Behälter (1) angebracht bzw. solche Führungsschienen (11) Bestandteil des Behälters (1).

Alternativ ist der Träger für die Hybridisierungslösung ein fester Bestandteil des Behälters, insbesondere eine Aussparung in dem Behälter.

Bei einer besonders bevorzugten Ausführungsform der erfindungsgemäßen Anordnung ist der Träger für die Hybridisierungslösung eine Wanne (3), insbesondere eine Wanne, die mit Aussparungen (12) zur Aufnahme von Flüssigkeit und/oder zur Aufnahme von mit Flüssigkeit getränkten Kissen versehen ist.

Die Materialien für sämtliche Bestandteile der Anordnung, ausgenommen den Objektträger, umfassen vorzugsweise Kunststoffe, besonders bevorzugt Polyethylen und/oder Polypropylen. Ferner können die Materialien für die vorstehend genannten Bestandteile der Anordnung auch Metalle umfassen.

Der Objektträger ist vorzugsweise aus Glas hergestellt, besonders bevorzugt aus Glas, das den hydrolytischen Klassen 1 bis 4 nach DIN 12111 entspricht.

Bei einem weiteren Aspekt der vorliegenden Erfindung wird ein Verfahren zum spezifischen Nachweis von Mikroorganismen durch in situ-Hybridisierung bereitgestellt, das die folgenden Schritte umfasst:
a) Fixieren der in einer Probe enthaltenen Mikroorganismen;
b) Inkubieren der fixierten Zellen mit nachweisbaren , Nukleinsäuresondenmolekülen;
c) Entfernen bzw. Abwaschen der nicht hybridisierten Nukleinsäuresondenmoleküle und
d) Detektieren der mit den Nukleinsäuresondenmolekülen hybridisierten Zellen,
wobei die Schritte a) bis c) und gegebenenfalls d) mit der erfindungsgemäßen in situ-Hybridisierungs-Anordnung durchgeführt werden.

Vorzugsweise werden die Fixierung und/oder gegebenenfalls Trocknungsschritte auf dem Objektträger durchgeführt.

Erfindungsgemäß bevorzugt ist es ferner, dass die abschließende Trocknung des Objektträgers in seitlicher Stellung des Objektträgers erfolgt und/oder die Inkubation bei horizontaler Stellung des Objektträgers erfolgt und/oder das Waschen bei vertikaler Stellung des Objektträgers erfolgt.

Bei einer weiteren bevorzugten Ausführungsform des erfindungsgemäßen Verfahrens wird in Schritt b) ein Gemisch aus einer Hybridisierungslösung und einer Nukleinsäurensondenmolekül-Lösung auf den Objektträger aufgebracht.

Besonders bevorzugt wird das vorstehend genannte Gemisch mittels eines Tropfgefäßes aufgebracht. Dieses Tropfgefäß ist bei einer weiteren bevorzugten Ausführungsform ein Einmal-Tropfgefäß oder ein Tropfgefäß für den mehrmaligen Gebrauch.

Die Hybridisierungslösung, die für die feuchte Kammer benötigt wird, kann bei dem erfindungsgemäßen Verfahren über sich in dem Träger für die Hybridisierungslösung befindende, mit Hybridisierungslösung getränkte Kissen in die erfindungsgemäße Anordnung eingeführt werden.

Vorzugsweise ist das in Schritt b) verwendete Nukleinsäurensondenmolekül komplementär zu einer chromosomalen oder episomalen DNA, einer mRNA oder rRNA eines nachzuweisenden Mikororganismus.

Erfindungsgemäß bevorzugt ist ferner, dass das Nukleinsäurensondenmolekül kovalent mit einem detektierbaren Marker verbunden ist. Dieser detektierbare Marker ist vorzugsweise ausgewählt aus der Gruppe der folgenden Marker:
- Fluoreszenzmarker,
- Chemolumineszenzmarker,
- radioaktiver Marker,
- enzymatisch aktive Gruppe,
- Hapten,
- durch Hybridisierung nachweisbare Nukleinsäure.

Vorzugsweise ist der Mikroorganismus bei dem erfindungsgemäßen Verfahren ein einzelliger Mikroorganismus. Besonders bevorzugt ist der Mikroorganismus eine Hefe, ein Bakterium, eine Alge oder ein Pilz. Bei einer weiteren bevorzugten Ausführungsform ist der Mikroorganismus ein Abwasserbakterium.

Bei weiteren Ausführungsformen des erfindungsgemäßen Verfahrens ist die Probe eine Umweltprobe und aus Wasser, Boden oder Luft entnommen; bzw. eine Lebensmittelprobe, insbesondere eine Probe aus Milch oder Milchprodukten, Trinkwasser, Getränken, Backwaren oder Fleischwaren; bzw. eine medizinische Probe, insbesondere eine aus Gewebe, Sekreten oder Stuhl gewonnene Probe; bzw. eine Probe aus Abwasser, insbesondere eine aus Belebtschlamm, Faulschlamm oder anaerobem Schlamm gewonnene Probe; bzw. eine aus einem Biofilm gewonnene Probe, insbesondere eine Probe, bei der der Biofilm aus einer industriellen Anlage gewonnen wird, bei der Abwasserreinigung gebildet wurde oder ein natürlicher Biofilm ist; bzw. eine einem pharmazeutischen oder kosmetischen Produkt entnommene Probe.

Erfindungsgemäß wird ferner ein Kit zum spezifischen Nachweis von Mikroorganismen durch in situ-Hybridisierung bereitgestellt, das mindestens ein Nukleinsäuresondenmolekül zum spezifischen Nachweis eines Mikroorganismus; mindestens eine Hybridisierungslösung; gegebenenfalls ein Nukleinsäuresondenmolekül zum Durchführen einer Negativkontrolle; gegebenenfalls ein Nukleinsäuresondenmolekül zum Durchführen einer Positivkontrolle; gegebenenfalls eine Waschlösung, gegebenenfalls eine Fixierungslösung, gegebenenfalls ein Antifading-Reagens sowie eine erfindungsgemäße in situ-Hybridisierungs-Anordnung umfaßt.

Vorzugsweise ist das Nukleinsäuresondenmolekül in dem erfindungsgemäßen Kit komplementär zu einer chromosomalen oder episomalen DNA, einer mRNA oder rRNA eines nachzuweisenden Mikroorganismus.

Erfindungsgemäß bevorzugt ist das Nukleinsäuresondenmolekül in dem erfindungsgemäßen Kit vorzugsweise kovalent mit einem detektierbaren Marker verbunden. Besonders bevorzugt ist der detektierbare Marker ausgewählt aus der Gruppe, bestehend aus Fluoreszenzmarkern, Chemolumineszenzmarkern, radioaktiven Markern, enzymatisch aktiven Gruppen, Haptenen und durch Hybridisierung nachweisbaren Nukleinsäuren.

Ein weiterer Gegenstand der vorliegenden Erfindung ist die Verwendung der erfindungsgemäßen in situ-Hybridisierungs-Anordnung zum spezifischen Nachweis von Mikroorganismen durch in situ-Hybridisierung.

Schließlich ist ein weiterer Gegenstand der vorliegenden Erfindung die Verwendung des erfindungsgemäßen Kits in dem erfindungsgemäßen Verfahren.

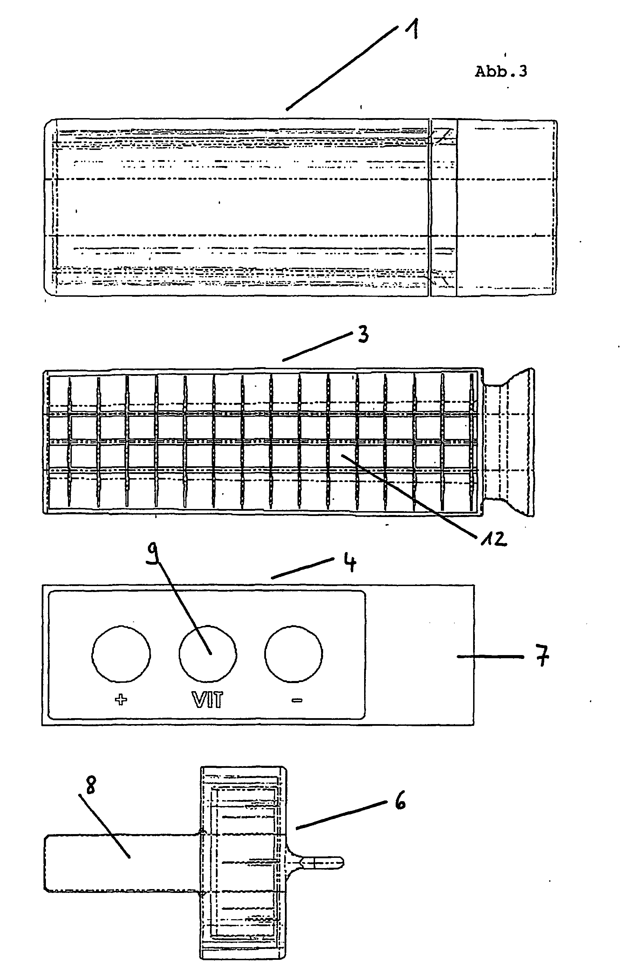

Es ist jetzt überraschenderweise gelungen, eine in situ-Hybridisierungs-Anordnung bereitzustellen, die eine schnelle und sichere Identifizierung von Mikroorganismen einfach und reproduzierbar ermöglicht. Die erfindungsgemäße Anordnung umfasst einen mit mindestens einer Öffnung versehenen Behälter, der im folgenden auch als Kammer bezeichnet wird; einen Träger für die Hybridisierungslösung, insbesondere eine Wanne, welche vollständig in den Behälter bzw. die Kammer eingebracht werden kann oder Teil dieses Behälters ist; sowie eine Befestigungsmöglichkeit für einen Objektträger. Ferner umfasst die Anordnung einen Objektträger, der zur in situ-Hybridisierung in die Kammer eingeführt werden kann. Der Aufbau einer erfindungsgemäßen Anordnung ist in Abbildung 3 dargestellt.

Vorzugsweise umfasst die erfindungsgemäße Anordnung ferner einen Deckel, der zum dichten Abschließen, insbesondere zum wasserdichten und/oder luftdichten Abschließen, einer Öffnung des Behälters geeignet ist. Der Objektträger wird vorzugsweise an diesem Deckel befestigt, besonders bevorzugt eingesteckt. Bei dieser bevorzugten Ausführungsform steckt der Objektträger fest und sicher im Deckel, kann aber zur abschließenden Auswertung per Hand und ohne größere Kraftanstrengung aus dem Deckel wieder ausgezogen werden. Die Fixierung bzw. die Befestigung des Deckels ermöglicht es, den Waschvorgang auch bei einer vertikalen Stellung der erfindungsgemäßen Anordnung sicher durchzuführen.

Bei einer besonders bevorzugten Ausführungsform der erfindungsgemäßen Anordnung ist der Deckel mit einem Bauteil versehen bzw. der Deckel umfasst ein Bauteil, welches eine stabile Standlage des Deckels mit befestigtem Objektträger getrennt von der Anordnung bei horizontaler bzw. vertikaler Stellung des Objektträgers ermöglicht. Diese Standfestigkeit des Deckels, die erfindungsgemäß beispielsweise durch das Anbringen eines Standbeins erreicht wurde, ermöglicht es, den Objektträger bei allen stattfindenden Reaktionen in einer horizontalen Lage zu halten.

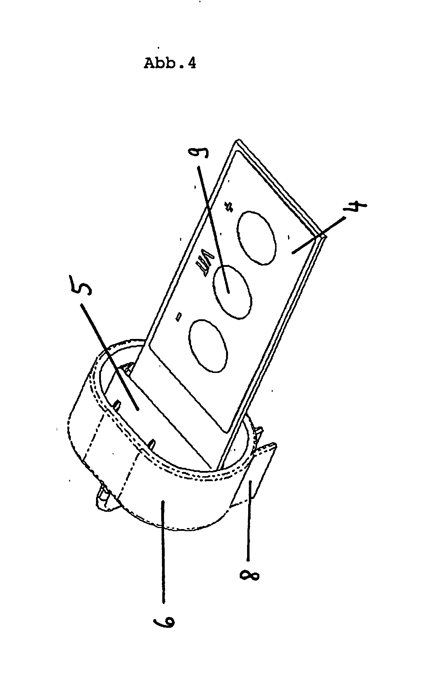

Der Objektträger ist zur leichteren Handhabung vorzugsweise am Deckel der in situ-Hybridisierungs-Anordnung befestigt (siehe Abbildung 4). In diesem Fall kann der Objektträger während des gesamten erfindungsgemäßen Verfahrens der Hybridisierung mit dem Deckel verbunden bleiben. Ferner sind dadurch alle vorbereitenden Prozeduren wie Waschen, Fixieren und dergleichen in der selben Reaktionskammer möglich. Das Versehen des Deckels mit einem Bauteil, welches eine stabile Standlage des Deckels getrennt von der Anordnung bei horizontaler bzw. vertikaler Stellung des Objektträgers ermöglicht, besitzt den wesentlichen Vorteil, dass der Objektträger sogar beim Auftragen der Proben und Sonden im Deckel belassen werden kann und gleichzeitig ein ebener und sicherer Stand beim Auftragen der Proben gewährleistet ist. Ferner ermöglicht das Bauteil bzw. das Standbein des Deckels, bei einer Befestigung des Objektträgers am Deckel auf diesem Objektträger die einzelnen Reaktionen zur Herbeiführung der Hybridisierungen von Nukleinsäuresonden mit Zellen durchzuführen. Ferner können mit einem derartigen Bauteil versehenen Deckel alle Trocknungsvorgänge auch außerhalb der Kammer durchgeführt werden. Durch den ebenen und sicheren Stand des Deckels mit befestigtem Objektträger kann einem Verfließen der Proben auf dem Objektträger vorgebeugt werden.

Bei einer weiteren bevorzugten Ausführungsform der vorliegenden Erfindung sind der Behälter und/oder der Deckel so konstruiert, dass bei geschlossenem Deckel eine stabile Standlage der Anordnung bei horizontaler Stellung des Objektträgers ermöglicht ist. Die horizontale Lagerung des Objektträgers insbesondere während der Schritte a) und b) des Verfahrens ist durch die Konstruktion der Auflageflächen der Bestandteile der erfindungsgemäßen in situ-Hybridisierungs-Anordnung somit gewährleistet.

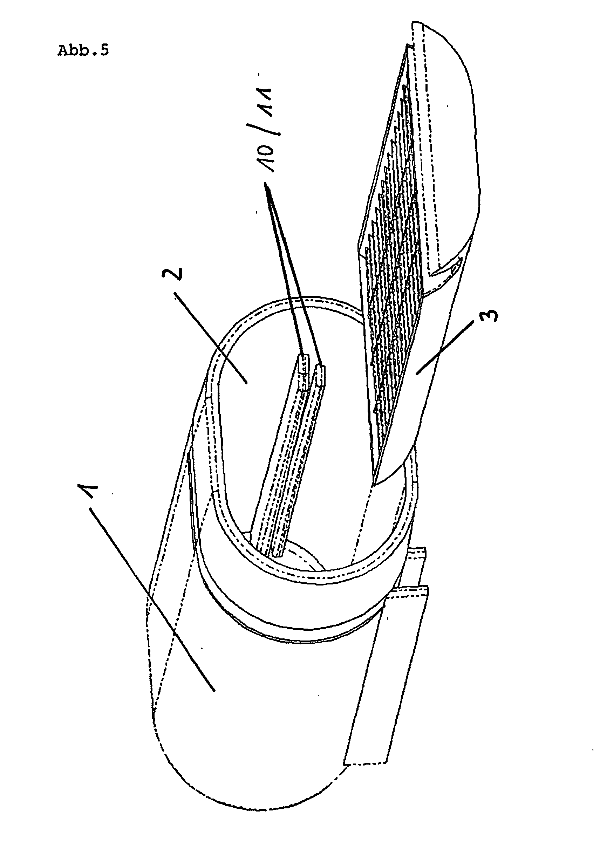

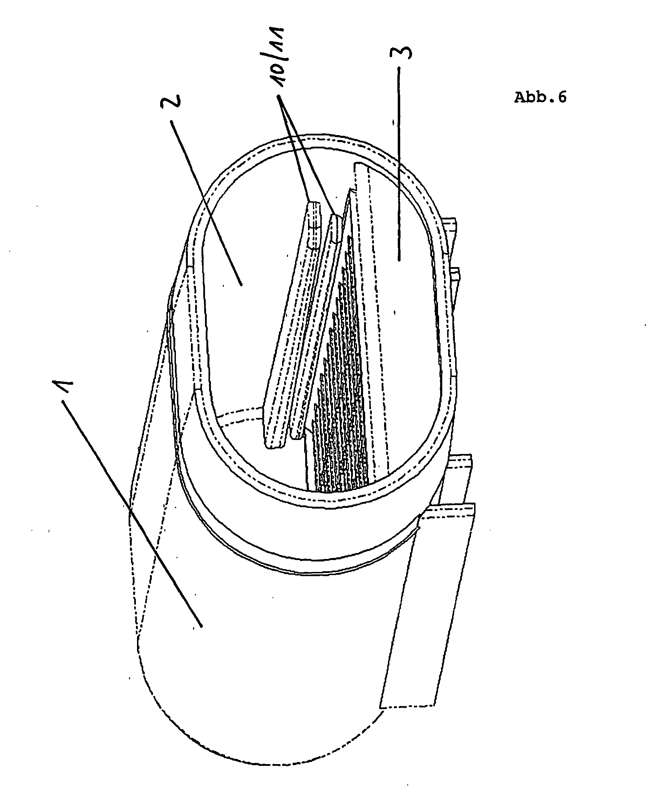

Die Verwendung einer Wanne als Träger für die Hybridisierungslösung ermöglicht, die Hybridisierungslösung, die für die feuchte Kammer benötigt wird, sicher und sauber in der erfindungsgemäßen Anordnung zu plazieren. Die Wanne besitzt vorzugsweise kleine Aussparungen zur Aufnahme von Flüssigkeit (siehe Abbildung 5). Besonders bevorzugt ist ferner, dass die Wanne zunächst nur ansatzweise in die Kammer gesteckt ist, so dass die Hybridisierungslösung, die für die Gewährleistung einer feuchten Kammer erforderlich ist, in die Wanne gegeben werden kann. Anschließend wird die Wanne vorzugsweise vollständig in die Kammer eingeschoben (siehe Abbildung 6).

Alternativ kann als Träger für die Hybridisierungslösung Zellstoff verwendet werden.

Die Konstruktion einer austauschbaren Wanne, in die die Hybridisierungslösung hineingetropft werden kann, und die nach der Verwendung entfernt werden kann, bietet den Vorteil, schnell eine definierte Menge an Hybridisierungslösung in die erfindungsgemäße in situ-Hybridisierungs-Anordnung einführen zu können. Das Verwenden von Zellstoff ist dabei nicht erforderlich, aber auch nicht ausgeschlossen.

Eine weitere Alternative zum Einbringen der Hybridisierungslösung in die erfindungsgemäße in situ-Hybridisierungs-Anordnung besteht darin, die Hybridisierungslösung über Einwegkissen, welche sich in der Wanne befinden, in den Reaktor einzubringen. Vorzugsweise sind die in Einwegkissen mit einer Frischeversiegelung versiegelt, die abgerissen wird, sobald sich die Wanne in der Kammer befindet, und die Hybridisierungslösung kann anschließend in der Kammer verdampfen.

Eine bevorzugte Ausführungsform für das in der erfindungsgemäßen in situ-Hybridisierungs-Anordnung durchgeführte Verfahren zur schnellen und einfachen Durchführung der in situ-Hybridisierung für die spezifische Analyse von Mikroorganismen umfasst die folgenden Schritte:
a) Fixieren der in einer Probe enthaltenen Mikroorganismen auf einem Objektträger;
b) Inkubation der fixierten Zellen mit Nukleinsäuresonden in der erfindungsgemäßen Anordnung zur Herbeiführung einer Hybridisierung;
c) Entfernen bzw. Abwaschen der nicht hybridisierten Oligonukleotide;
d) Detektieren der mit den Oligonukleotiden hybridisierten Zellen.

Sowohl die Inkubation als auch der Waschvorgang finden vorzugsweise in der erfindungsgemäßen in situ-Hybridisierungs-Anordnung statt.

Vorzugsweise werden die Mikroorganismen nicht wie üblicherweise zuerst in einem Reaktionsgefäß fixiert und anschließend immobilisiert, sondern die Fixierung und/oder gegebenenfalls die Trocknung finden direkt auf dem Objektträger statt.

Eine derartige Fixierung auf dem Objektträger vermeidet Zellverluste und ist wesentlich einfacher in der Handhabung und unkomplizierter in der Durchführung. Zudem ermöglicht die Fixierung auf dem Objektträger den Fixierungsschritt sowie die Dehydratisierungsreihe in einem Arbeitsdurchgang zu vereinen.

Die Hybridisierung und Zugabe der Nukleinsäuresondenmoleküle erfolgt erfindungsgemäß vorzugsweise nicht, indem wie üblicherweise zuerst eine definierte Menge einer Hybridisierungslösung und dann eine definierte Menge einer Sondenlösung in eine Objektträgeraussparung mittels einer Pipette pipettiert wird, sondern indem ein Gemisch aus einer Hybridisierungs- und einer Nukleotidsondenmolekül-Lösung auf den Objektträger aufgebracht wird.

Das Auftragen eines Gemisches aus Hybridisierungs- und Nukleinsäuresondenmolekül-Lösung ermöglicht bei gleicher Stabilität der Nukleinsäuresondenmoleküle eine schnellere und fehlerlose Verfahrensweise.

Vorzugsweise wird das vorstehend genannte Gemisch über ein Tropfgefäß durch leichten Druck tropfenweise aufgebracht. Das Tropfgefäß kann entweder für den mehrmaligen Gebrauch bestimmt sein und mehrere Tropfen der Mischlösung aus der Hybridisierungs- und der Sondenlösung enthalten oder alternativ ein kleines Einmal-Tropfgefäß sein, das unter Beachtung eines Totvolumens die erforderliche Reagenzienmenge enthält.

Die Verwendung von Tropfgefäßen erspart die Verwendung von teuren Pipetten und erleichtert ferner die Handhabung und Dosierung.

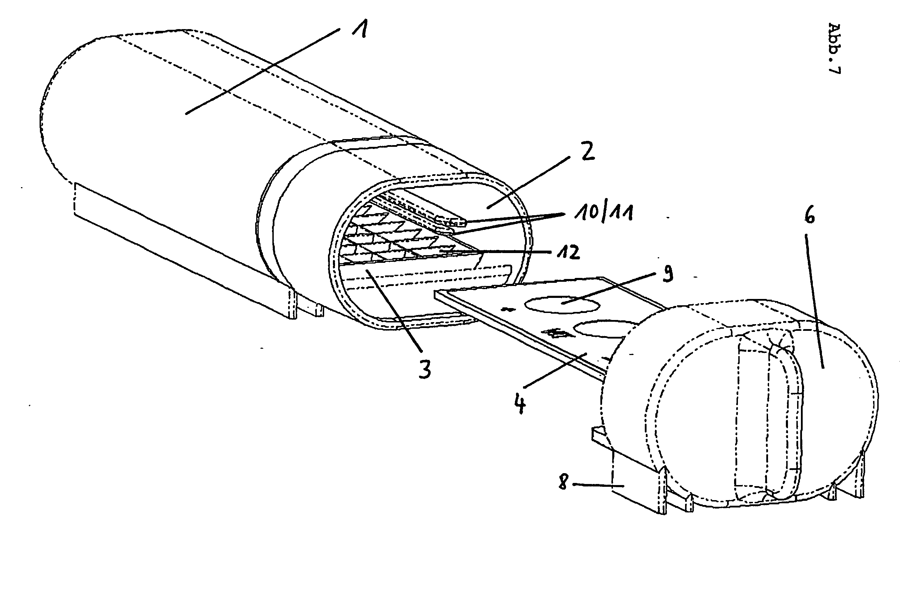

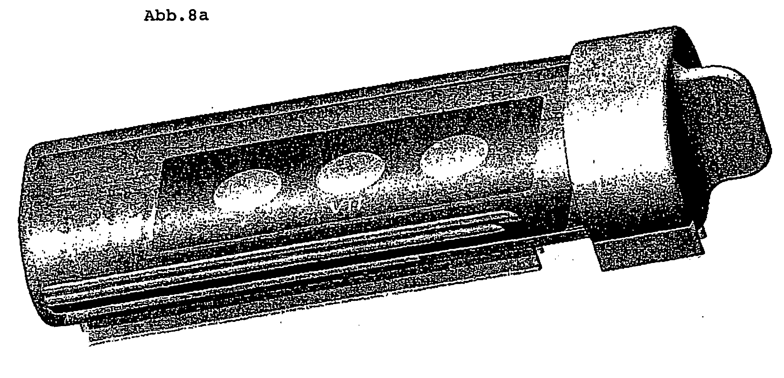

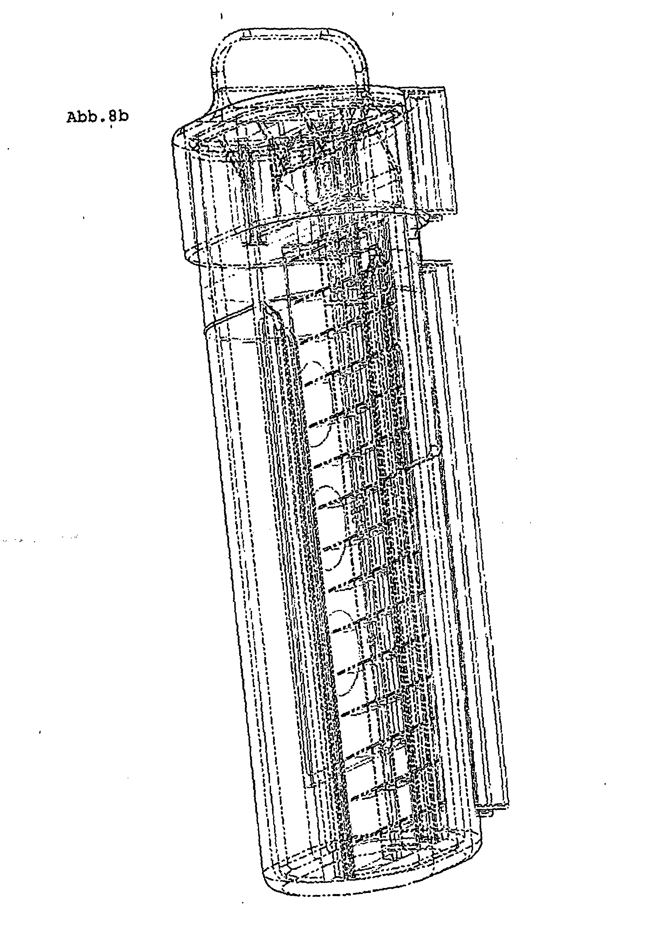

Anschließend wird der vorzugsweise an dem Deckel befestigte Objektträger in die Kammer hineingeführt (siehe Abbildung 7). In der Kammer vorzugsweise angebrachte seitliche Führungslinien sorgen hierbei für eine problemlose Einführung und weitere Fixierung bzw. Stabilisierung des Objektträgers in der Kammer (siehe Abbildung 8).

Vorzugsweise ist die Kammer und/oder der Deckel derart konstruiert, dass eine stabile horizontale wie vertikale wie seitliche Standlage gewährleistet ist.

Die anschließende Inkubation erfolgt vorzugsweise in horizontaler Stellung des Objektträgers. Das anschließende Waschen des Objektträgers erfolgt erfindungsgemäß vorzugsweise in der Kammer und besonders bevorzugt in vertikaler Stellung des Objektträgers.

Zur abschließenden Trocknung und insbesondere zur abschließenden Lufttrocknung des Objektträgers ist der Deckel vorzugsweise so konstruiert, dass er seitlich hingestellt werden kann. Dies ist insbesondere deshalb vorteilhaft, da so die Tropfen vom Objektträger ablaufen können, ohne in eine andere Aussparung des Objektträgers hineinzulaufen.

Folglich werden vorzugsweise die meisten Verfahrensschritte in ein und demselben Gefäß bestehend aus Kammer und vorzugsweise Deckel der Anordnung durchgeführt.

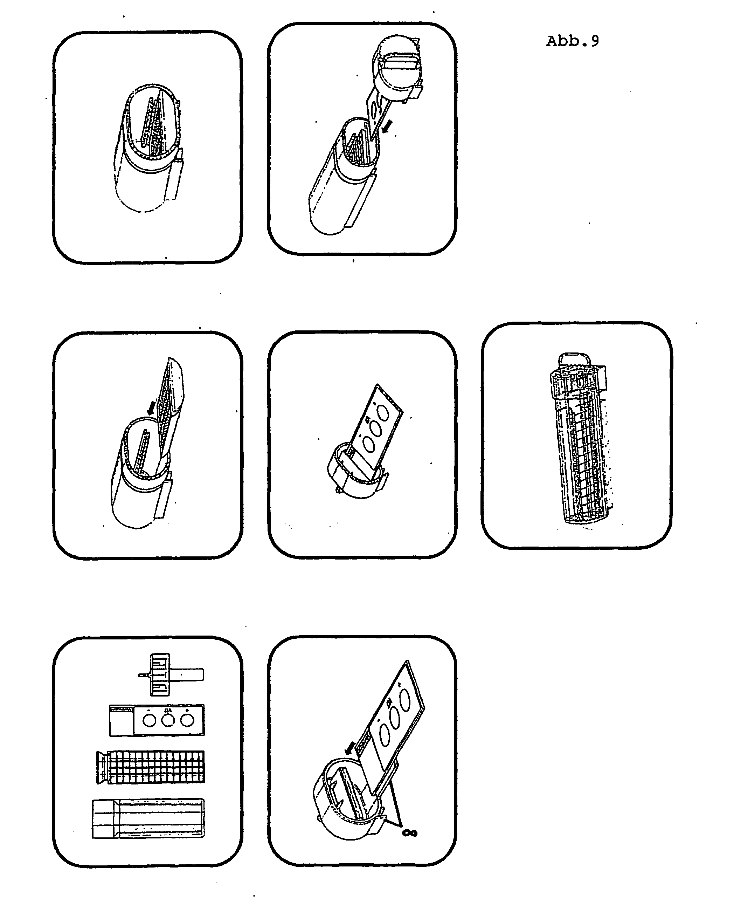

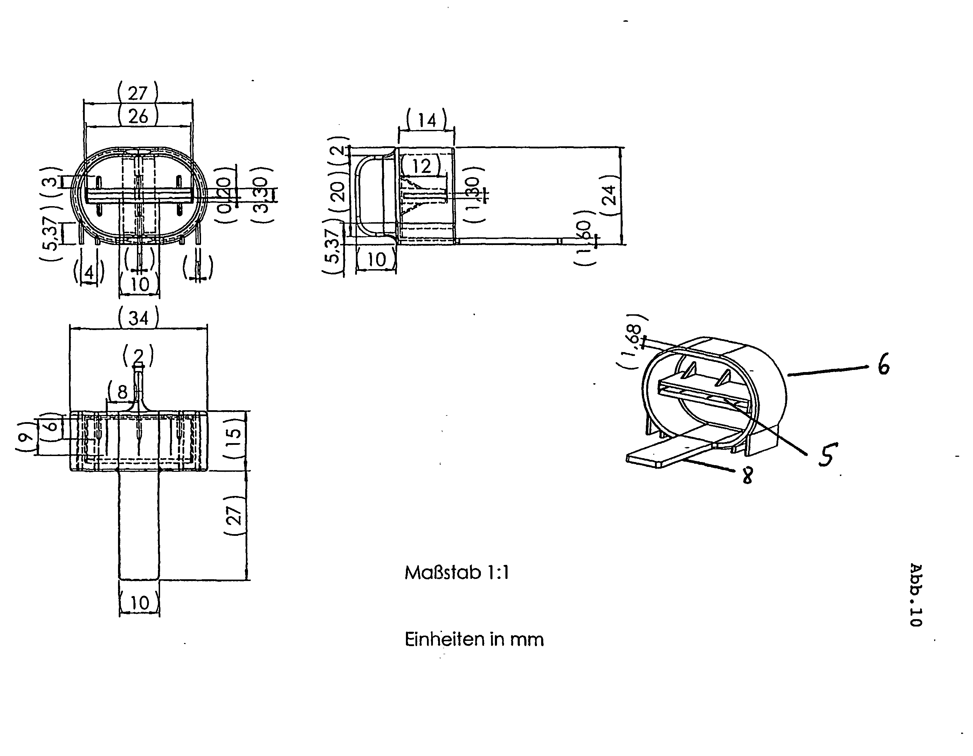

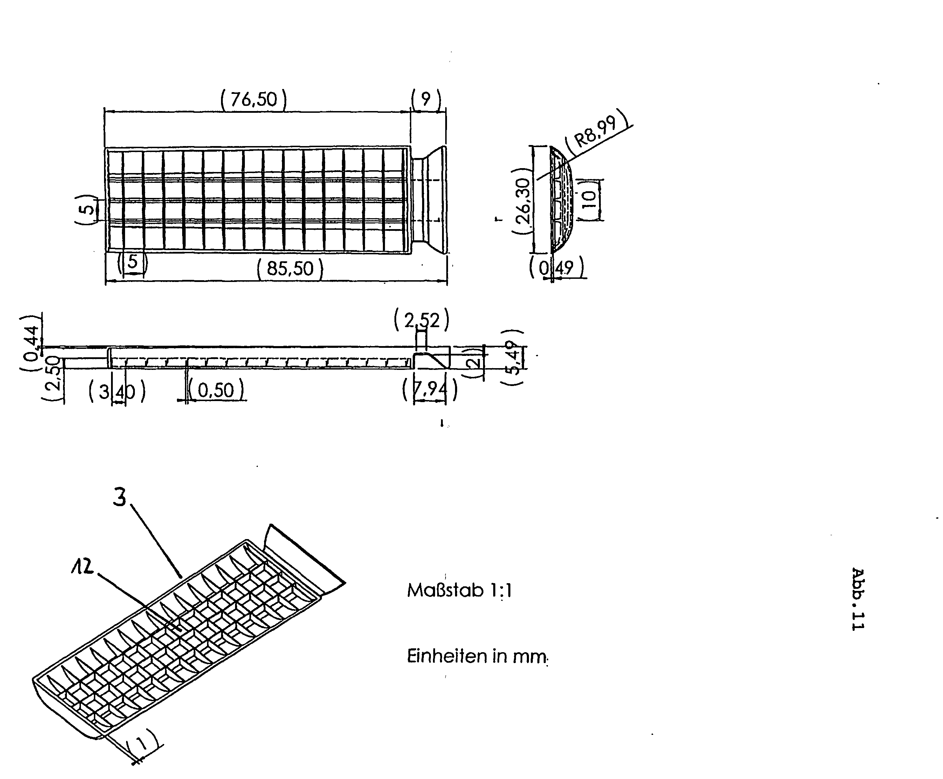

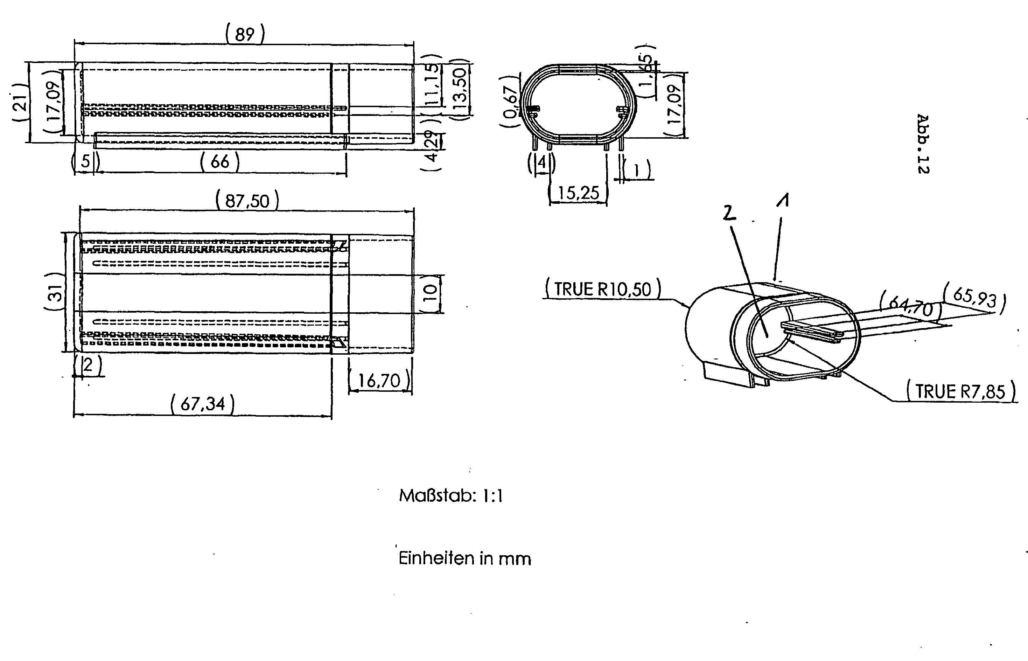

In Abbildung 9 ist der gesamte Aufbau einer bevorzugten Ausführungsform der erfindungsgemäßen in situ-Hybridisierungs-Anordnung, umfassend Deckel, Kammer, Wanne und zugeführten Objektträger dargestellt. Die Abbildungen 10 bis 12 zeigen die Abmessungen von Deckel, Wanne und Kammer.

Wesentliche Vorteile der erfindungsgemäßen in situ-Hybridisierungs-Anordnung sowie des erfindungsgemäßen Verfahrens zum spezifischen Nachweis von Mikroorganismen gegenüber den herkömmlichen Verfahren der in situ-Hybridisierung und insbesondere den herkömmlichen FISH-Verfahren sind somit die sehr leichte Handhabung sowie die Schnelligkeit und Reproduzierbarkeit, mit der der spezifische Nachweis von Mikroorganismen in einer Probe möglich ist.

Im Rahmen der vorliegenden Erfindung wird unter "Fixieren" von Mikroorganismen eine Behandlung verstanden, mit der die Zellhülle der Mikroorganismen für Nukleinsäuresonden durchlässig gemacht wird. Die Nukleinsäuresonden, die aus einem Oligonukleotid und einem daran gebundenen Marker bestehen, können dann die Zellhülle penetrieren und an die der Nukleinsäuresonde entsprechenden Zielsequenz im Zellinneren binden. Die Bindung ist als eine Ausbildung von Wasserstoffbrücken zwischen komplementären Nukleinsäurestücken zu verstehen. Bei der Hülle kann es sich um eine Lipidhülle handeln, die einen Virus umgibt, um die Zellwand von Bakterien oder um die Zellmembran eines einzelligen Eukaryonten. Zur Fixierung wird üblicherweise Ethanol verwendet. Kann die Zellwand mit diesen Maßnahmen nicht für Nukleinsäuresonden penetrierbar gemacht werden, so sind dem Fachmann ausreichend weitere Maßnahmen bekannt, die zu demselben Ergebnis führen. Dazu zählen beispielsweise eine niederprozentige Paraformaldehydlösung oder eine verdünnte Formaldehydlösung, Methanol, Mischungen von Alkoholen, enzymatische Behandlungen oder ähnliches.

Bei der Nukleinsäuresonde im Sinne der Erfindung kann es sich um eine DNA- oder RNA-Sonde handeln, die in der Regel zwischen 12 und 1000 Nukleotide umfassen wird, bevorzugt zwischen 12 und 50, besonders bevorzugt zwischen 17 und 25 Nukleotiden. Die Auswahl der Nukleinsäuresonden geschieht nach den Gesichtspunkten, ob eine komplementäre Sequenz in dem nachzuweisenden Mikroorganismus vorliegt. Durch diese Auswahl einer definierten Sequenz, kann dadurch eine Bakterienart, eine Bakteriengattung oder eine ganze Bakteriengruppe erfaßt werden. Komplementarität muß bei einer Sonde von 12 bis 15 Nukleotiden Länge über 100% der Sequenz gegeben sein. Bei Oligonukleotiden mit mehr als 15 Nukleotiden sind ein bis mehrere Fehlpaarungsstellen erlaubt. Durch das Einhalten von stringenten Hybridisierungsbedingungen wird gewährleistet, dass das Nukleinsäuresondenmolekül auch tatsächlich mit der Zielsequenz hybridisiert. Moderate Bedingungen im Sinne der Erfindung sind z.B. 0% Formamid in einer Hybridisierungslösung wie sie in Beispiel 1 beschrieben ist. Stringente Bedingungen im Sinne der Erfindung sind beispielsweise 20-80% Formamid in der Hybridisierungslösung.

Die Dauer der Hybridisierung beträgt üblicherweise zwischen 10 Minuten und 12 Stunden; bevorzugt erfolgt die Hybridisierung für etwa 2 Stunden. Die Hybridisierungstemperatur beträgt bevorzugt zwischen 44 °C und 48 °C, besonders bevorzugt 46 °C, wobei der Parameter der Hybridisierungstemperatur, wie auch die Konzentration an Salzen und Detergentien in der Hybridisierungslösung in Abhängigkeit von der Sonde bzw. den Sonden, insbesondere deren Länge(n) und dem Grad der Komplementarität zur Zielsequenz in der nachzuweisenden Zelle optimiert werden kann. Der Fachmann ist mit hier einschlägigen Berechnungen vertraut.

Im Rahmen des erfindungsgemäßen Verfahrens hat eine typische Hybridisierungslösung eine Salzkonzentration von 0,1 M bis 1,5 M, bevorzugt von 0,9 M, wobei es sich bei dem Salz vorzugsweise um Natriumchlorid handelt. Weiter umfaßt die Hybridisierungslösung üblicherweise ein Detergens, wie z.B. Natriumdodecylsulfat (SDS), in einer Konzentration von 0,001-0,1%, bevorzugt in einer Konzentration von 0,0 1 %, und Tris/HCl in einem Konzentrationsbereich von 0,001-0,1 M, vorzugsweise in einer Konzentration von 0,02 M. Der pH-Wert von Tris/HCl beträgt üblicherweise zwischen 6 und 10, wobei ein pH von ca. 8,0 bevorzugt ist. Wie oben erwähnt, kann die Hybridisierungslösung des weiteren zwischen 0% und 80% Formamid enthalten, je nachdem welcher Grad von Stringenz erwünscht ist bzw. benötigt wird.

Die Nukleinsäuresonde sollte in der Hybridisierungslösung wenn möglich in einer Menge von 15 ng bis 1000 ng anwesend sein, wobei diese Menge in einem Volumen an Hybridisierungslösung zwischen 8 µl und 100 µl, vorzugsweise in 40 µl, enthalten ist. Besonders bevorzugt beträgt die Sondenkonzentration 111 ng/40 µl Hybridisierungslösung.

Nach erfolgter Hybridisierung sollten die nicht hybridisierten und überschüssigen Sondenmoleküle entfernt werden, was üblicherweise mittels einer herkömmlichen Waschlösung bzw. eines herkömmlichen Waschpuffers erfolgt. Diese Waschlösung kann, falls gewünscht, 0,001-0,1% eines Detergens wie SDS, wobei eine Konzentration von 0,01 % bevorzugt wird, sowie Tris/HCl in einer Konzentration von 0,001-0,1 M, bevorzugt 0,02 M, enthalten, wobei der pH-Wert von Tris/HCl im Bereich von 6,0 bis 10,0, vorzugsweise bei 8,0 liegt. Ein Detergens kann enthalten sein, ist aber nicht zwingend erforderlich. Weiter enthält die Waschlösung üblicherweise NaCl, wobei die Konzentration je nach benötigter Stringenz von 0,003 M bis 0,9 M, bevorzugt von 0,01 M bis, 0,9 M, beträgt. Des weiteren kann die Waschlösung EDTA enthalten, wobei die Konzentration vorzugsweise 0,005 M beträgt. Ferner kann die Waschlösung auch dem Fachmann geläufige Konservierungsmittel in geeigneten Mengen enthalten.

Das "Abwaschen" der nicht gebundenen Sondenmoleküle erfolgt üblicherweise bei einer Temperatur im Bereich von 44 °C bis 52 °C, bevorzugt von 44°C bis 50°C und besonders bevorzugt bei 46 °C für eine Dauer von 10-40 Minuten, vorzugsweise für 15 Minuten.

Die Auswahl der jeweiligen Nukleinsäuresonden erfolgt in Abhängigkeit vom nachzuweisenden Mikroorganismus. Die Nukleinsäuresonde kann dabei komplementär zu einer chromosomalen oder episomalen DNA sein, aber auch zu einer mRNA oder rRNA des nachzuweisenden Mikroorganismus. Von Vorteil ist es, eine Nukleinsäuresonde zu wählen, die zu einem Bereich komplementär ist, der in einer Kopiezahl von mehr als 1 im nachzuweisenden Mikroorganismus vorliegt. Die nachzuweisende Sequenz liegt bevorzugt 500 - 100000 mal pro Zelle vor, besonders bevorzugt 1000 - 50000 mal. Aus diesem Grunde wird bevorzugt die rRNA als Zielstelle verwendet, da die Ribosomen in der Zelle, als Orte der Proteinbiosynthese vieltausendfach in jeder aktiven Zelle vorliegen.

Erfindungsgemäß wird die Nukleinsäuresonde mit dem im obengenannten Sinne fixierten Mikroorganismus inkubiert, um so ein Eindringen der Nukleinsäuresondenmoleküle in den Mikroorganismus und die Hybridisierung von Nukleinsäuresondenmolekülen mit den Nukleinsäuren des Mikroorganismus zu erlauben. Anschließend werden die nicht-hybridisierten
Nukleinsäuresondenmoleküle durch übliche Waschschritte entfernt. Die spezifisch hybridisierten Nukleinsäuresondenmoleküle können anschließend in den jeweiligen Zellen detektiert werden.

Voraussetzung sowohl für die Identifizierung als auch für die Quantifizierung ist, daß das erfindungsgemäß verwendete Nukleinsäuresondenmolekül nachweisbar ist. Diese Nachweisbarkeit kann z.B. durch kovalente Verbindung des Nukleinsäuresondenmoleküls mit einem detektierbaren Marker sichergestellt werden. Als detektierbare Marker werden fluoreszierende Gruppen wie z.B. CY2, CY3, CY5, FITC, FLUOS, TRITC oder FLUOS-PRIME verwendet, die dem Fachmann alle wohlbekannt sind. Beispiele für fluoreszierende Gruppen sind in der nachfolgenden Tabelle 1 angegeben:

**TABELLE 1**

| | |
|---|---|
| FLUOS | 5,(6)-Carboxyfluorescein-N-hydroxysuccinimedester (Boehringer Mannheim, Mannheim, Deutschland); ε = 7,50 x 104 mol⁻¹ l⁻¹, Absₘₐₓ bei 494 nm; Emₘₐₓ bei 518nm, MG = 473. |
| TRITC | Tetramethylrhodamin-5,6 isothiocyanat (Isomer G. Molecular Probes Inc., Eugene, USA, Lambda, Graz, AT); ε = 1,07 x 105 mol⁻¹ l⁻¹, Absₘₐₓ bei 537nm; Emₘₐₓ bei 566 nm, MG = 479. |
| CT | 5,(6)-Carboxytetramethylrhodamin-N-hydroxysuccinimidester |
| | (Molecular Probes Inc., Eugene, USA); ε = 0,87 x 105 mol⁻¹ l⁻¹, Absₘₐₓ bei 537 nm; Emₘₐₓ bei 566 nm. |
| CY-3 | NHS-Ester von Cy5.18 (Biological Detection Systems, Pittsburgh, USA);(Amersham Life Sciences, Inc., Arlington Heights, USA); ε = 1,5 x 105mol⁻¹ l⁻¹, Absₘₐₓ bei 532 nm; Emₘₐₓ bei 565 nm. MG = 765,95. |
| CY-5 | NHS-Ester von Cy5.18 (Biological Detection Systems, Pittsburgh, USA); (Amersham Life Sciences, Inc., Arlington Heights, USA); ε = > 2 x 105 mol⁻¹ l⁻¹, Absₘₐₓ bei 650 nm; Emₘₐₓ bei 667 nm. MG = 791,99. |

Alternativ werden chemolumineszierende Gruppen oder radioaktive Markierungen, z.B. ³⁵S, ³²P, ³³P, ¹²⁵J, verwendet. Nachweisbarkeit kann aber auch gegeben sein durch Kopplung des Nukleinsäuresondenmoleküls mit einem enzymatisch aktiven Molekül, beispielsweise alkalischer Phosphatase, saurer Phosphatase, Peroxidase, Meerrettichperoxidase, β-D-Galaktosidase oder Glukoseoxidase. Für jedes dieser Enzyme ist eine Reihe von Chromogenen bekannt, die anstelle des natürlichen Substrats umgesetzt werden können, und entweder zu farbigen oder zu fluoreszierenden Produkten umgesetzt werden können. Beispiele für solche Chromogene sind in der nachfolgenden Tabelle 2 angegeben.

**TABELLE 2**

| Enzyme | Chromogen |
|---|---|
| 1. Alkalische Phosphatase und saure Phosphatase | 4-Methylumbelliferylphosphat (*), Bis(4-Methyiumbelliferylphosphat), (*) 3-O-Methylfluoreszein, Flavon-3-Diphosphattriammoniumsalz (*), p-Nitrophenylphosphatdinatriumsalz |
| | |
| 2. Peroxidase | Tyraminhydrochlorid (*), 3-(p-Hydroxyphenyl)-Propionsäure (*), p-Hydroxyphenethylalkohol (*), 2,2'-Azino-di-3-ethylbenzthiazolinsulfon säure (ABTS), ortho-Phenylendiamindihydrochlorid, o-Dianisidin, 5-Aminosalicylsäure, p-Ucresol (*), 3,3'-dimethyloxybenzidin, 3-Methyl-2-benzothiazolinhydrazon, Tetramethylbenzidin |
| | |
| 3. Meerrettichperoxidase | H₂O₂ + Diammoniumbenzidin H₂O₂ + Tetramethylbenzidin |
| | |
| 4. β-D-Galaktosidase | o-Nitrophenyl-β-D-galaktopyranosid, 4-Methylumbelliferyl-β-D-galaktosid |
| 5. Glukoseoxidase | ABTS, Glukose und Thiazolylblau |

| | |
|---|---|
| * Fluoreszenz | |

Schließlich ist es möglich, die Nukleinsäuresondenmoleküle so zu gestalten, dass an ihrem 5'- oder 3'-Ende eine weitere zur Hybridisierung geeignete Nukleinsäuresequenz vorhanden ist. Diese Nukleinsäuresequenz umfasst wiederum ca. 12 bis 1000, bevorzugt 15 - 50 Nukleotide. Dieser zweite Nukleinsäurebereich kann wiederum von einer Oligonukleotidsonde erkannt werden, die durch eines der oben erwähnten Mittel nachweisbar ist.

Eine weitere Möglichkeit besteht in der Kopplung der nachweisbaren Nukleinsäuresondenmoleküle mit einem Hapten. Nach Ablösung der Nukleinsäuresondenmoleküle von der Zielnukleinsäure können die nunmehr separat vorliegenden Nukleinsäuresondenmoleküle mit das Hapten erkennenden nachweisbaren Antikörpern in Kontakt gebracht werden. Ein bekanntes Beispiel für ein solches Hapten ist Digoxigenin oder seine Derivate. Dem Fachmann sind über die angegebenen Beispiele hinaus viele weitere Möglichkeiten bekannt, ein zur Hybridisierung verwendetes Oligonukleotid zu detektieren und zu quantifizieren.

Die Vielzahl möglicher Markierungen ermöglicht auch den gleichzeitigen Nachweis von zwei oder mehreren sich überlappenden oder auch nicht überlappenden Populationen. So können z.B. durch die Verwendung von zwei oder mehr verschiedenen Fluoreszenzmarkern mehrere Bakterienpopulationen nachgewiesen werden (R. Amann, J. Snaidr, M. Wagner, W. Ludwig, and K.-H. Schleifer. 1996. In situ visualization of high genetic diversity in a natural microbial community. J. Bacteriol. 178:12, 3496-3500).

Die Auswertung hängt von der Art der Markierung der verwendeten Sonde ab. Im Rahmen der vorliegenden Erfindung kann die Auswertung vorteilhaft mit einem Lichtmikroskop, Epifluoreszenz-Mikroskop, Chembluminometer, Fluorometer und dergleichen erfolgen.

Der mittels des erfindungsgemäßen Verfahrens nachzuweisende Mikroorganismus kann ein prokaryontischer oder eukaryontischer Mikroorganismus sein. In den meisten Fällen wird es erwünscht sein, einzellige Mikroorganismen nachzuweisen. Diese einzellige Mikroorganismen können auch in größeren Aggregaten, sogenannten Filamenten, vorliegen. Relevante Mikroorganismen sind hierbei vor allem Hefen, Algen, Bakterien oder Pilze.

In einer besonders bevorzugten Ausführungsform der vorliegenden Erfindung handelt es sich bei den Mikroorganismen um Bakterien, die im Abwasser von Abwasserreinigungsanlagen vorkommen.

Das erfindungsgemäße Verfahren kann vielfältig angewendet werden.
Umweltproben können auf das Vorhandensein von Mikroorganismen untersucht werden. Diese Proben können hierzu aus Luft, Wasser oder aus dem Boden entnommen sein.

Ein weiteres Anwendungsgebiet für das erfindungsgemäße Verfahren ist die Kontrolle von Lebensmitteln. In bevorzugten Ausführungsformen werden die Lebensmittelproben aus Milch oder Milchprodukten (Joghurt, Käse, Quark, Butter, Buttermilch), Trinkwasser, Getränken (Limonaden, Bier, Säfte), Backwaren oder Fleischwaren entnommen. Für den Nachweis von Mikroorganismen in Lebensmitteln kann u.U. eine Kultivierung notwendig sein, um sicherzustellen, dass Mikroorganismen in ausreichender Menge vorliegen.

Das erfindungsgemäße Verfahren kann weiter zur Untersuchung medizinischer Proben eingesetzt werden. Es ist für die Untersuchung von Gewebeproben, z.B.
Biopsiematerial aus der Lunge, Tumor- oder entzündliches Gewebe, aus Sekreten wie Schweiß, Speichel, Sperma und Ausfluss aus der Nase, Harnröhre oder Vagina sowie für Urin- oder Stuhlproben geeignet.

Ein weiteres Anwendungsgebiet für das vorliegende Verfahren ist die Untersuchung von Abwässern, z.B. Belebtschlamm, Faulschlamm oder anaeroben Schlamm. Darüber hinaus ist es geeignet, Biofilme in industriellen Anlagen zu analysieren, sowie auch sich natürlicherweise bildende Biofilme oder bei der Abwasserreinigung bildende Biofilme zu untersuchen. Auch die Untersuchung pharmazeutischer und kosmetischer Produkte, z.B. Salben, Cremes, Tinkturen, Säfte etc. ist mit dem erfindungsgemäßen Verfahren möglich.

Erfindungsgemäß wird bei einem weiteren Aspekt der vorliegenden Erfindung ein Kit zur Durchführung des Verfahrens zum Nachweis von Mikroorganismen in einer Probe bereitgestellt. Der Inhalt eines solchen Kits richtet sich im wesentlichen nach der Natur des nachzuweisenden Mikroorganismus. Er umfasst als wichtigsten Bestandteil eine oder mehrere für den jeweils nachzuweisenden Mikroorganismus spezifische Nukleinsäuresonde(n) sowie bevorzugt weitere Nukleinsäuresonden, mit denen eine Negativ- bzw. Positivkontrolle durchgeführt werden kann. Darüber hinaus umfasst er vorzugsweise eine Hybridisierungslösung und eine Waschlösung. Die Wahl der Hybridisierungslösung hängt in erster Linie von der Länge der verwendeten Nukleinsäuresonden ab. So müssen, wie dem Fachmann bekannt ist, für die Hybridisierung einer Nukleinsäuresonde von 15 Nukleotiden weniger stringente Bedingungen gewählt werden als für die Hybridisierung einer Sonde von 75 Nukleotiden Länge. Beispiele für Hybridisierungsbedingungen sind z.B. in Stahl & Amann (1991) in Stackebrand u. Goodfellow (Hrsg.), Nucleic Acid Techniques in Bacterial Systematics; John Wiley & Sons Ltd., Chichester, UK, angegeben.

Erfindungsgemäß wird somit ferner ein Kit bereitgestellt, mit dem das vorstehend beschriebene erfindungsgemäße Verfahren durchgeführt werden kann. Der erfindungsgemäße Kit umfasst in einer bevorzugten Ausführungsform mindestens ein Nukleinsäuresondenmolekül zum spezifischen Nachweis eines Mikroorganismus; mindestens eine Hybridisierungslösung; gegebenenfalls ein Nukleinsäuresondenmolekül zum Durchführen einer Negativkontrolle; gegebenenfalls ein Nukleinsäuresondenmolekül zum Durchführen einer Positivkontrolle; gegebenenfalls eine Waschlösung; gegebenenfalls eine Fixierungslösung, gegebenenfalls ein Antifading-Reagens; sowie die erfindungsgemäße in situ-Hybridisierungs-Anordnung, wobei in der Anordnung oder in Teilen der Anordnung folgende Schritte durchführbar sind:
a) Fixieren der in einer Probe enthaltenen Mikroorganismen auf einem Objektträger;
b) Inkubation der fixierten Zellen mit Nukleinsäuresonden, um eine Hybridisierung herbeizuführen;
c) Entfernen bzw. Abwaschen der nicht hybridisierten Nuklotidsondenmoleküle.

In einer bevorzugten Ausführungsform enthält der Kit spezifische Sonden zum Nachweis von Bakterien, die im Abwasser von Abwasserreinigungsanlagen vorkommen.

Durch das erfindungsgemäße Verfahren kann die in situ-Hybridisierung in der Praxis etabliert werden.

Die folgenden Beispiele und Abbildungen dienen der Erläuterung der Erfindung und sollen nicht in einschränkender Weise ausgelegt werden.

### Beispiel: Nachweis von Bakterien in einer Abwasserprobe

### 1. Allgemeine Beschreibung

Das nachfolgende Beispiel des erfindungsgemäßen Verfahrens, im folgenden auch "VIT-Verfahren" genannt, in der erfindungsgemäßen in situ-HybridisierungsAnordnung, im folgenden auch "VIT-Reaktor" genannt, dient zur qualitativen Analyse von Bakterien, die in Abwasserproben vorkommen. Die Identifizierung erfolgt in wenigen Stunden.

### 2. Grundprinzip

Die Bakterien werden durch dieses Verfahren mit fluoreszenzmarkierten Oligonukleotidsonden hybridisiert und können anschließend mit einem Epifluoreszenz-Mikroskop auf dem Objektträger nachgewiesen werden.

### 3. Zubehör

Trockenschrank, vorgeheizt auf 46°C
Flasche zum Ansetzen und Erwärmen der Waschlösung
Messzylinder zum Ansetzen der Waschlösung
Thermometer
Wecker
VIT-Lösung: Lösung mit spezifischen Nukleinsäuresondenmolekülen
Negative Control: Lösung für Negativkontrolle
Positive Control: Lösung für Positivkontrolle
Solution A und B: Fixierungslösung
Solution C: Hybridisierungslösung '
Solution D: Waschlösung
Finisher: Antifading Reagenz

Objektträger mit drei Aussparungen (1 Aussparung für die eigentliche Hybridisierung, mit VIT gekennzeichnet; 1 Aussparung für die Negativkontrolle, mit - gekennzeichnet; 1 Aussparung für die Positivkontrolle, mit + gekennzeichnet) Deckgläser

### 4. Durchführung

Vor dem Beginn einer Analyse den Trockenschrank auf 46 °C vorheizen.

Auftragen der Proben und Fixierung
1. Objektträger in den Deckel des VIT-Reaktors einsetzen.
2. je 1 Tropfen Probenmaterial in die drei Aussparungen des Objektträgers überführen, Objektträger (ohne VIT-Reaktor) waagerecht inkubieren (46°C, 30 min oder bis vollständig trocken).
3. je 1 Tropfen "Solution A" in jede Aussparung auf dem Objektträger auftropfen, Objektträger (ohne VIT-Reaktor) waagerecht inkubieren (46°C, 30 min oder bis vollständig trocken).
4. je 1 Tropfen "Solution B" in jede Aussparung auf dem Objektträger auftropfen, Objektträger (ohne VIT-Reaktor) waagerecht inkubieren (Raumtemperatur, 1 min oder bis vollständig trocken).

### Hybridisierung

5. 1 Tropfen "Negative Control" auf die mit "-" gekennzeichnete Aussparung des Objektträgers auftropfen.
6. 1 Tropfen "Positive Control" auf die mit "+" gekennzeichnete Aussparung des Objektträgers auftropfen.
7. 1 Tropfen "VIT" auf die mit "VIT" gekennzeichnete Aussparung des Objektträgers auftropfen.
8. Wanne halb in den VIT-Reaktor einsetzen.
9. etwa 20-30 Tropfen "Solution C" in die Wanne des VIT-Reaktors tropfen, Wanne ganz in den Reaktor einschieben.
10. Objektträger vorsichtig waagerecht in den VIT-Reaktor einschieben, VIT-Reaktor schließen und waagerecht inkubieren (46°C, 1.5h).
ACHTUNG: Die einzelnen Tropfen dürfen NICHT ineinander laufen.
11. Waschlösung vorbereiten.
pro VIT-Reaktor werden 25 ml Waschlösung benötigt. Hierzu wird "Solution D" zehnfach mit destilliertem Wasser verdünnt. In der Tabelle 3 sind Verdünnungs-Beispiele angegeben.

**Tabelle 1**

| | Mengen in ml für | | |
|---|---|---|---|
| | 1 VIT-Reaktor | 3 VIT-Reaktoren | 10 VIT-Reaktoren |
| Solution D | 2,5 | 7,5 | 25 |
| Destilliertes Wasser | 22,5 | 67,5 | 225 |
| Endvolumen | 25 | 75 | 250 |

12. Die fertige Waschlösung während der Hybridisierung in einem geschlossenen Gefäß im Trockenschrank auf 46°C vorwärmen.

### Waschen

13. VIT-Reaktor vorsichtig öffnen und Objektträger entnehmen.
14. ACHTUNG: Die einzelnen Tropfen dürfen NICHT ineinander laufen.
15. VIT-Reaktor aufstellen und bis zur Markierung mit der vorgewärmten (s. Schritt 5.2.7) Waschlösung füllen. Objektträger senkrecht einstellen, VIT-Reaktor schließen und senkrecht inkubieren (46 °C, 15 min).
16. VIT-Reaktor öffnen und Objektträger entnehmen. Waschlösung ausgießen.
17. VIT-Reaktor bis zur Markierung mit destilliertem Wasser füllen. Objektträger senkrecht einstellen, und sofort anschließend wieder entnehmen, Wasser ausgießen.
18. Objektträger vertikal stellen und inkubieren (46°C, 15 min oder bis vollständig trocken)
19. Zwischen die Aussparungen des Objekträgers je 1Tropfen "Finisher" geben und das Deckglas auflegen.

### Abbildungen

### Abbildung 1:

Darstellung eines Sekundärstrukturmodells der 16S rRNA

### Abbildung 2:

Schematische Darstellung der FISH-Technik. Während der in situ-Hybridisierung dringen verschiedenartig markierte Sonden A und B in die Zellen A und C ein. Die Zelle A besitzt ribosomale Nukleinsäuren mit den Bindungsstellen für Sonden des Typs A, nicht aber für Sonden des Typs B und kann folglich Sonden vom Typ B nicht binden. Zelle C besitzt weder Bindungsstellen für Sonde A noch für Sonde B und kann deshalb keine der beiden Sonden binden. Nach dem anschließenden Waschschritt befinden sich lediglich gebundene Sonden in den Zellen. Zelle A kann nun auf Grund ihrer Fluoreszenz im Fluoreszenzmikroskop detektiert werden.

### Abbildung 3:

Draüfsicht auf die Bestandteile einer,besonderen Ausführungsform der erfindungsgemäßen in situ-Hybridisierungs-Anordnung: Behälter (1), Wanne (3), Objektträger (4), Deckel (6) mit Standbein (8) (von links nach rechts)

### Abbildung 4:

Schematische Darstellung einer besonders bevorzugten Ausführungsform des Deckels (6) mit Schlitz (5) zur Befestigung des Objektträgers (4) und Standbein (8) sowie des Objektträgers (4).

### Abbildung 5:

Schematische Darstellung einer bevorzugten Ausführungsform der erfindungsgemäßen in situ-Hybridisierungs-Anordnung. Die Wanne (3) besitzt kleine Aussparungen zur Aufnahme von Flüssigkeit und wird zunächst nur ansatzweise in die Kammer (1) gesteckt, um die Hybridisierungslösung, die für eine feuchte Kammer erforderlich ist, in die Wanne (3) geben zu können.

### Abbildung 6:

Schematische Darstellung einer besonderen Ausführungsform der erfindungsgemäßen in situ-Hybridisierungs-Anordnung mit vollständig eingeschobener Wanne (3).

### Abbildung 7:

Schematische Darstellung einer besonders bevorzugten Ausführungsform der erfindungsgemäßen in situ-Hybridisierungs-Anordnung, in die der an einem Deckel (6) befestigte Objektträger (4) eingeführt wird.

### Abbildung 8:

Schematische Darstellung einer bevorzugten Ausführungsform der erfindungsgemäßen in situ-Hybridisierungs-Anordnung. In der Kammer befindliche seitliche Führungslinien sorgen für eine problemlose Einführung und weitere Fixierung bzw. Stabilisierung des Objektträgers sowie der Wanne in der Kammer. Sowohl die Kammer als auch der Deckel besitzen eine Konstruktion, die eine stabile horizontale wie senkrechte Standlage erlauben.
a) Schematisierte Perspektivansicht, bei der zum besseren Verständnis Teile der Anordnung transparent dargestellt sind.
b) Umrißdarstellung von a).

### Abbildung 9:

Schematische Darstellung des Zusammenbaus der einzelnen Komponenten der erfindungsgemäßen in situ-Hybridisierungs-Anordnung zum spezifischen Nachweis von Mikroorganismen durch in situ-Hybridisierung unter Verwendung der erfindungsgemäßen in situ-Hybridisierungs-Anordnung.

### Abbildung 10:

Maßstabsgerechte Zeichnung einer bevorzugten Ausführungsform des Deckels (6).

### Abbildung 11:

Maßstabsgerechte Zeichnung einer bevorzugten Ausführungsform der Wanne (3).

### Abbildung 12:

Maßstabsgerechte Zeichnung einer bevorzugten Ausführungsform des Behälters (1) bzw. der Kammer (1).

## Patentansprüche

1. In situ-Hybridisierungs-Anordnung zum spezifischen Nachweis von Mikroorganismen in einer Probe, umfassend:
- einen mit mindestens einer Öffnung (2) versehenen Behälter (1);
- einen Träger für die Hybridisierungslösung (3);
- einen Objektträger (4); und
- einen Deckel (6), der die Befestigungsmittel (5) für den Objektträger (4) umfasst und zum dichten Abschließen der Öffnung (2) des Behälters (1) geeignet ist.

2. Anordnung nach Anspruch 1,
wobei der Deckel (6) als Befestigungsmittel (5) für den Objektträger (4) einen Schlitz (5) umfasst, in den ein Ende (7) des Objektträgers (4) gesteckt werden kann.

3. Anordnung nach Anspruch 1 oder 2,
wobei der Deckel (6) mit einem Bauteil (8) versehen ist, welches eine stabile Standlage des Deckels (6) mit befestigtem Objektträger (4) getrennt von der Anordnung ohne Deckel (6) bei horizontaler Stellung des Objektträgers (4) ermöglicht.

4. Anordnung nach einem der vorangehenden Ansprüche,
wobei der Deckel (6) so konstruiert ist, dass er das Stehen des Deckels (6) mit befestigtem Objektträgers (4) getrennt von der Anordnung ohne Deckel (6) bei horizontaler bzw. vertikaler bzw. seitlicher Stellung des Objektträgers (4) ermöglicht.

5. Anordnung nach einem der vorangehenden Ansprüche,
wobei der Behälter (1) und/oder der Deckel (6) so konstruiert sind, dass sowohl bei nicht geschlossenem Deckel (6) als auch bei geschlossenem Deckel (6) eine stabile Standlage dei Anordnung bei horizontaler bzw. vertikaler bzw. seitlicher Stellung des Objektträgers (4) möglich ist.

6. Anordnung nach einem der vorangehenden Ansprüche,
wobei der Objektträger (4) mit Aussparungen (9) versehen ist, in die die zu untersuchende Probe und gegebenenfalls Negativ- bzw. Positivproben getrennt voneinander aufgetragen werden können.

7. Anordnung nach einem der vorangehenden Ansprüche,
wobei die Aussparungen (9) auf dem Objektträger (4) nur in einer Dimension von weiteren Aussparungen (9) benachbart sind.

8. Anordnung nach einem der vorangehenden Ansprüche,
wobei seitliche Führungsschienen (10) für den Objektträger (4) an dem Behälter (1) zur Stabilisierung des Objektträgers (4) in dem Behälter (1) angebracht sind.

9. Anordnung nach einem der vorangehenden Ansprüche,
wobei der Träger für die Hybridisierungslösung (3) herausnehmbar bzw. einsetzbar ist.

10. Anordnung nach einem der vorangehenden Ansprüche,
wobei der Träger für die Hybridisierungslösung (3) vollständig in den Behälter (1) eingebracht werden kann.

11. Anordnung nach einem der vorangehenden Ansprüche,
wobei der Träger für die Hybridisierungslösung (3) teilweise in den Behälter (1) eingebracht werden kann.

12. Anordnung nach einem der vorangehenden Ansprüche,
wobei seitliche Führungsschienen (11) für den Träger für die Hybridisierungslösung (3) an dem Behälter (1) zur Stabilisierung des Trägers für die Hybridisierungslösung (3) in dem Behälter (1) angebracht sind.

13. Anordnung nach einem der Ansprüche 1 bis 8,
wobei der Träger für die Hybridisierungslösung (3) ein fester Bestandteil des Behälters (1) ist.

14. Anordnung nach Anspruch 13,
wobei der Träger für die Hybridisierungslösung (3) eine Aussparung in dem Behälter (1) ist.

15. Anordnung nach einem der vorangehenden Ansprüche,
wobei der Träger für die Hybridisierungslösung (3) eine Wanne (3) ist, insbesondere eine Wanne (3), die mit Aussparungen (12) zur Aufnahme von Flüssigkeit und/oder zur Aufnahme von mit Flüssigkeit getränkten Kissen versehen ist.

16. Anordnung nach einem der vorangehenden Ansprüche,
wobei die Materialien für sämtliche Bestandteile der Anordnung, ausgenommen den Objektträger (4), Kunststoffe, insbesondere Polyethylen und/oder Polypropylen, umfassen.

17. Anordnung nach einem der vorangehenden Ansprüche,
wobei der Objektträger (4) aus Glas, insbesondere aus Glas, das den hydrolytischen Klassen 1 bis 4 nach DIN 12111 entspricht, hergestellt ist.

18. Verfahren zum spezifischen Nachweis von Mikroorganismen in einer Probe durch in situ-Hybridisierung, umfassend die folgenden Schritte:
a) Fixieren der in der Probe enthaltenen Mikroorganismen;
b) Inkubieren der fixierten Zellen mit nachweisbaren Nukleinsäuresondenmolekülen;
c) Entfernen bzw. Abwaschen der nicht hybridisierten Nukleinsäuresondenmoleküle; und
d) Detektieren der mit den Nukleinsäuresondenmolekülen hybridisierten Zellen,
wobei die Schritte a) bis c) und gegebenenfalls d) mit der in situ-Hybridisierungs-Anordnung nach einem der Ansprüche 1 bis 17 durchgeführt werden.

19. Verfahren nach Anspruch 18,
wobei die Fixierung und/oder gegebenenfalls Trocknungsschritte auf dem Objektträger (4) durchgeführt werden.

20. Verfahren nach Anspruch 18 oder 19,
wobei die Trocknung, insbesondere die abschließende Trocknung des Objektträgers (4) in seitlicher Stellung des Objektträgers (4) erfolgt.

21. Verfahren nach einem der Ansprüche 18 bis 20,
wobei die Inkubation bei horizontaler Stellung des Objektträgers (4) erfolgt.

22. Verfahren nach einem der Ansprüche 18 bis 21,
wobei das Waschen bei vertikaler Stellung des Objektträgers (4) erfolgt.

23. Verfahren nach einem der Ansprüche 18 bis 22,
wobei in Schritt b) des Verfahrens ein Gemisch aus einer Hybridisierungslösung und einer Nukleinsäuresondenmolekül-Lösung auf den Objektträger (4) aufgebracht wird.

24. Verfahren nach Anspruch 23,
wobei das Gemisch mittels eines Tropfgefäßes aufgebracht wird.

25. Verfahren nach Anspruch 24,
wobei das Tropfgefäß ein Einmal-Tropfgefäß oder ein Tropfgefäß für den mehrmaligen Gebrauch ist.

26. Verfahren nach einem der Ansprüche 18 bis 23,
wobei die Hybridisieruhgslösung über sich in dem Träger für die Hybridisierungslösung (3) befindende, mit Hybridisierungslösung getränkte Kissen in die Anordnung nach einem der Ansprüche 1 bis 17 eingeführt wird.

27. Kit zum spezifischen Nachweis von Mikroorganismen durch in situ-Hybridisierung, umfassend:
- mindestens ein Nukleinsäuresondenmolekül zum spezifischen Nachweis eines Mikroorganismus;
- mindestens eine Hybridisierungslösung;
- gegebenenfalls ein Nukleinsäuresonderunolekül zum Durchführen einer Negativkontrolle;
- gegebenenfalls ein Nukleinsäuresondenmolekül zum Durchführen einer Positivkontrolle;
- gegebenenfalls eine Waschlösung;
- gegebenenfalls eine Fixierungslösung; und
- eine in situ-Hybridisierungs-Anordnung nach einem der Ansprüche 1 bis 17.

28. Verwendung der Anordnung nach einem der Ansprüche 1 bis 17 zum spezifischen Nachweis von Mikroorganismen durch in situ-Hybridisierung.

29. Verwendung des Kits nach Anspruch 27 in dem Verfahren nach einem der Ansprüche 18 bis 26.

## Claims

1. In situ hybridisation arrangement for specific detection of micro-organisms in a sample, comprising:
- a container (1) provided with at least one opening (2);
- a support for the hybridisation solution (3);
- a specimen slide (4); and
- a cover (6), which comprises the securing means (5) for the specimen slide (4) and is suitable for sealed closure of the opening (2) of the container (1).

2. An arrangement according to claim 1,
wherein the cover (6) comprises as securing means (5) for the specimen slide (4) a slot (5) into which one end (7) of the specimen slide (4) may be inserted.

3. An arrangement according to claim 1 or claim 2,
wherein the cover (6) is provided with a component (8) which makes it possible for the cover (6) with specimen slide (4) secured therein to stand stably separately from the arrangement without its cover (6), keeping the specimen slide (4) in a horizontal position.

4. An arrangement according to any one of the preceding claims,
wherein the cover (6) is so constructed that it makes it possible for the cover (6) with the specimen slide (4) secured therein to stand separately from the arrangement without its cover (6) with the specimen slide (4) in a horizontal, vertical or sideways position.

5. An arrangement according to any one of the preceding claims,
wherein the container (1) and/or the cover (6) are so constructed that it is possible for the arrangement to adopt a stable standing position with the specimen slide (4) in a horizontal, vertical or sideways position both with the cover (6) open and with the cover (6) closed.

6. An arrangement according to any one of the preceding claims,
wherein the specimen slide (4) is provided with recesses (9), into which the sample to be investigated and optionally negative or positive samples may be applied separately from one another.

7. An arrangement according to any one of the preceding claims,
wherein the recesses (9) in the specimen slide (4) are adjacent other recesses (9) only in one dimension.

8. An arrangement according to any one of the preceding claims,
wherein lateral guide rails (10) for the specimen slide (4) are attached to the container (1) to stabilise the specimen slide (4) in the container (1).

9. An arrangement according to any one of the preceding claims,
wherein the support for the hybridisation solution (3) may be taken out or inserted.

10. An arrangement according to any one of the preceding claims,
wherein the support for the hybridisation solution (3) may be introduced fully into the container (1).

11. An arrangement according to any one of the preceding claims,
wherein the support for the hybridisation solution (3) may be introduced partially into the container (1).

12. An arrangement according to any one of the preceding claims,
wherein lateral guide rails (11) for the support for the hybridisation solution (3) are attached to the container (1) to stabilise the support for the hybridisation solution (3) in the container (1).

13. An arrangement according to any one of claims 1 to 8,
wherein the support for the hybridisation solution (3) is a fixed component of the container (1).

14. An arrangement according to claim 13,
wherein the support for the hybridisation solution (3) is a recess in the container (1).

15. An arrangement according to any one of the preceding claims,
wherein the support for the hybridisation solution (3) is a trough (3), in particular a trough (3) which is provided with recesses (12) for accommodating liquid and/or for accommodating liquid-impregnated pads.

16. An arrangement according to any one of the preceding claims,
wherein the materials for all the components of the arrangement, except the specimen slide (4), comprise plastics, in particular polyethylene and/or polypropylene.

17. An arrangement according to any one of the preceding claims,
wherein the specimen slide (4) is made of glass, in particular of glass which corresponds to hydrolytic classes 1 to 4 according to DIN 12111.

18. A method for specific detection of micro-organisms in a sample by in situ hybridisation, comprising the following steps:
a) immobilisation of the micro-organisms contained in the sample;
b) incubating the immobilised cells with detectable nucleic acid probe molecules;
c) removing or washing off the non-hybridised nucleic acid probe molecules; and
d) detecting the cells hybridised with the nucleic acid probe molecules,
wherein steps a) to c) and optionally d) are performed using the in situ hybridisation arrangement according to any one of claims 1 to 17.

19. A method according to claim 18,
wherein the immobilisation and/or optional drying steps are performed on the specimen slide (4).

20. A method according to claim 18 or claim 19,
wherein drying, in particular the final drying of the specimen slide (4) takes place with the specimen slide (4) in a sideways position.

21. A method according to any one of claims 18 to 20,
wherein incubation takes place with the specimen slide (4) in a horizontal position.

22. A method according to any one of claims 18 to 21,
wherein washing takes place with the specimen slide (4) in a vertical position.

23. A method according to any one of claims 18 to 22,
wherein, in step b) of the method, a mixture of a hybridisation solution and a nucleic acid probe molecule solution is applied to the specimen slide (4).

24. A method according to claim 23,
wherein the mixture is applied by means of a dropping vessel.

25. A method according to claim 24,
wherein the dropping vessel is a disposable dropping vessel or a dropping vessel for multiple use.

26. A method according to any one of claims 18 to 23,
wherein the hybridisation solution is introduced via pads located in the support for the hybridisation solution (3) and impregnated with hybridisation solution into the arrangement according to any one of claims 1 to 17.

27. A kit for specific detection of micro-organisms by in situ hybridisation, comprising
- at least one nucleic acid probe molecule for specific detection of a micro-organism;
- at least one hybridisation solution;
- optionally a nucleic acid probe molecule for performing a negative control;
- optionally a nucleic acid probe molecule for performing a positive control;
- optionally a washing solution;
- optionally an immobilising solution; and
- an in situ hybridisation arrangement according to any one of claims 1 to 17.

28. Use of the arrangement according to any one of claims 1 to 17 for specific detection of micro-organisms by in situ hybridisation.

29. Use of the kit according to claim 27 in the method according to any one of claims 18 to 26.

## Revendications

1. Dispositif d'hybridation *in situ* pour la détection spécifique de micro-organismes dans un échantillon, comprenant :
- un récipient (1) doté d'au moins une ouverture (2);
- un support pour la solution d'hybridation (3) ;
- un support pour objet (4) ; et
- un couvercle (6), qui comprend des moyens de fixation (5) pour le support pour objet (4) et qui est approprié à la fermeture étanche de l'ouverture (2) du récipient (1).

2. Dispositif selon la revendication 1, dans lequel le couvercle (6) comprend, à titre de moyens de fixation (5) pour le support pour objet (4), une fente (5), dans laquelle une extrémité (7) du support pour objet (4) peut être insérée.

3. Dispositif selon la revendication 1 ou 2, dans lequel le couvercle (6) est doté d'une pièce (8), qui permet une position stable du couvercle (6), avec le support pour objet (4) fixé, séparément du dispositif dont le couvercle (6) a été ôté, quand le support pour objet (4) se trouve en position horizontale.

4. Dispositif selon l'une des revendications précédentes, dans lequel le couvercle (6) est ainsi fait qu'il permet la position debout du couvercle (6), avec le support pour objet (4) fixé, séparément du dispositif dont le couvercle (6) a été ôté, quand le support pour objet (4) se trouve en position horizontale ou verticale ou latérale.

5. Dispositif selon l'une des revendications précédentes, dans lequel le récipient (1) et/ou le couvercle (6) sont ainsi faits qu'une position stable du dispositif est possible, aussi bien couvercle (6) fermé que couvercle (6) ouvert, quand le support pour objet (4) se trouve en position horizontale ou verticale ou latérale.

6. Dispositif selon l'une des revendications précédentes, dans lequel le support pour objet (4) est doté de cavités (9), dans lesquelles peuvent être déposés, séparément les uns des autres, l'échantillon et le cas échéant les échantillons positifs ou négatifs à analyser.

7. Dispositif selon l'une des revendications précédentes, dans lequel les cavités (9) sur le support pour objet (4) sont adjacentes aux autres cavités (9) uniquement dans une dimension.

8. Dispositif selon l'une des revendications précédentes, dans lequel des rails de guidage latéraux (10) destinés au support pour objet (4) sont aménagés sur le récipient (1) pour stabiliser le support pour objet (4) dans le récipient (1).

9. Dispositif selon l'une des revendications précédentes, dans lequel le support pour la solution d'hybridation (3) est amovible ou insérable.

10. Dispositif selon l'une des revendications précédentes, dans lequel le support pour la solution d'hybridation (3) peut être entièrement inséré dans le récipient (1).

11. Dispositif selon l'une des revendications précédentes, dans lequel le support pour la solution d'hybridation (3) peut être partiellement inséré dans le récipient (1).

12. Dispositif selon l'une des revendications précédentes, dans lequel des rails de guidage latéraux (11) destinés au support pour la solution d'hybridation (3) sont aménagés sur le récipient (1) pour stabiliser le support pour la solution d'hybridation (3) dans le récipient (1).

13. Dispositif selon l'une des revendications 1 à 8, dans lequel le support pour la solution d'hybridation (3) fait partie intégrante du récipient (1).

14. Dispositif selon la revendication 13, dans lequel le support pour la solution d'hybridation (3) est une cavité dans le récipient (1).

15. Dispositif selon l'une des revendications précédentes, dans lequel le support pour la solution d'hybridation (3) est un bac (3), en particulier un bac (3) qui est doté de cavités (12) pour accueillir le liquide et/ou pour accueillir des tampons imbibés de liquides.

16. Dispositif selon l'une des revendications précédentes, dans lequel les matériaux de tous les composants du dispositif, à l'exception du support pour objet (4), comprennent des matières plastiques, en particulier le polyéthylène et/ou le polypropylène.

17. Dispositif selon l'une des revendications précédentes, dans lequel le support pour objet (4) est fabriqué en verre, en particulier en un verre qui correspond aux classes hydrolytiques 1 à 4 d'après la norme DIN 12111.

18. Procédé pour la détection spécifique de micro-organismes dans un échantillon par hybridation *in situ,* comprenant les étapes suivantes :
a) fixation des micro-organismes contenus dans l'échantillon ;
b) incubation des cellules fixées avec des sondes d'acides nucléiques détectables ;
c) élimination ou lavage des sondes d'acides nucléiques non hybridées ; et
d) détection des cellules hybridées avec les sondes d'acides nucléiques,
les étapes a) à c) et le cas échéant d) étant réalisées avec le dispositif d'hybridation *in situ* selon l'une des revendications 1 à 17.

19. Procédé selon la revendication 18, où la fixation et/ou le cas échéant les étapes de séchage sont effectuées sur le support pour objet (4).

20. Procédé selon la revendication 18 ou 19, où le séchage, en particulier le séchage final du support pour objet (4), se fait en position latérale du support pour objet (4).

21. Procédé selon l'une des revendications 18 à 20, où l'incubation se fait en position horizontale du support pour objet (4).

22. Procédé selon l'une des revendications 18 à 21, où le lavage se fait en position verticale du support pour objet (4).

23. Procédé selon l'une des revendications 18 à 22, où, à l'étape b) du procédé, un mélange constitué d'une solution d'hybridation et d'une solution de sondes d'acides nucléiques est déposé sur le support pour objet (4).

24. Procédé selon la revendication 23, dans lequel le mélange est déposé à l'aide d'un goutte à goutte.

25. Procédé selon la revendication 24, dans lequel le goutte à goutte est un goutte à goutte jetable ou un goutte à goutte à usage multiple.

26. Procédé selon l'une des revendications 18 à 23, où la solution d'hybridation est introduite dans le dispositif selon l'une des revendications 1 à 17, par le biais de tampons imbibés de solution d'hybridation, se trouvant dans le support pour la solution d'hybridation (3).

27. Kit pour la détection spécifique de micro-organismes par hybridation *in situ*, comprenant :
- au moins une sonde d'acides nucléiques pour la détection spécifique d'un micro-organisme ;
- au moins une solution d'hybridation ;
- le cas échéant, une sonde d'acides nucléiques pour réaliser un contrôle négatif ;
- le cas échéant, une sonde d'acides nucléiques pour réaliser un contrôle positif ;
- le cas échéant, une solution de lavage ;
- le cas échéant, une solution de fixation ; et
- un dispositif d'hybridation *in situ* selon l'une des revendications 1 à 17.

28. Utilisation du dispositif selon l'une des revendications 1 à 17 pour la détection spécifique de micro-organismes par hybridation *in situ*.

29. Utilisation du kit selon la revendication 27 dans le procédé selon l'une des revendications 18 à 26.
